(19) Europäisches Patentamt
European Patent Office
Office européen des brevets

(11) EP 3 811 926 A1

(12) EUROPEAN PATENT APPLICATION
published in accordance with Art. 153(4) EPC

(43) Date of publication:
**28.04.2021 Bulletin 2021/17**

(21) Application number: **19796818.3**

(22) Date of filing: **24.04.2019**

(51) Int Cl.:
***A61K 8/97*** *(2017.01)* ***A61Q 17/00*** *(2006.01)*
***A61Q 19/00*** *(2006.01)*

(86) International application number:
**PCT/ES2019/070280**

(87) International publication number:
**WO 2019/211501 (07.11.2019 Gazette 2019/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **30.04.2018 ES 201830426**

(71) Applicant: **Monteloeder, S.L.**
**03203 Elche (Alicante) (ES)**

(72) Inventors:
- **CATURLA CERNUDA, Nuria**
  **03203 Elche (ES)**
- **PERAL CLEMENT, Ana**
  **03203 Elche (ES)**

(74) Representative: **Martin Alvarez, Juan Enrique**
**Ibidem Abogados Estrategas SLP**
**B54357488**
**Juan de la Cierva, 43, Planta 2 Local 1-1**
**03203 Elche (Alicante) (ES)**

# (54) COMPOSITION COMPRISING VEGETABLE EXTRACTS WITH FLAVONOIDS FOR ALLEVIATING THE MANY EFFECTS OF AIR POLLUTION ON THE SKIN

(57) The present invention provides a composition for its use in the treatment of the air pollution effects on the skin or for the premature skin aging associated with the air pollution effects. Said composition comprises diterpenes, oleuropein, verbascoside, quercetin and hydroxytyrosol. In a preferred embodiment the composition contains around 32 % weight of rosemary extract with a minimum diterpene content of 15 % weight, around 30 % weight of a olive fruits or leaves extract with a minimum oleuropein content of 16 % weight and a minimum hydroxytyrosol content of 5% weight, around 28 % weight of a *Lippia citriodora* extract with a minimum verbascoside content of 25 % weight and around 10 % weight of a *Sophora japonica* extract with a minimum quercetin content of 40 % weight.



Printed by Jouve, 75001 PARIS (FR)

## Description

### TECHNICAL FIELD OF THE INVENTION

The present invention belongs to the technical field of products for the treatment of human skin affections, in particular, for alleviating the multiple urban air pollution effects on the skin. Nevertheless, the present composition, in spited being specifically formulated to alleviate the multiple urban environmental pollution effects, can also be commercialised and used with different indications with notable alleviating effects, like, for example, as skin deterioration retarding product due to cellular aging.

### BACKGROUND OF THE INVENTION

Chronic exposure to urban air pollution causes simultaneous multiple harmful effects on human skin. Among these harmful effects are premature skin aging, allergic or inflammatory conditions such as the appearance of atopic skins, dermatitis, eczema, psoriasis or acne, even causing skin cancer, among the more serious affections that pollution can induce (1).

The connection between environmental pollution with the harmful effects it causes on the skin has been proven in numerous scientific studies during recent years.

For example, it has been scientifically demonstrated that several air pollutants, such as tobacco smoke, volatile organic compounds (VOC's), formaldehyde, toluene, nitrogen dioxide and particulate matter, act as risk factors for the development or worsening of atopic dermatitis, which is a skin inflammatory process (2, 3, 4). A possible explanation is that these air pollutants induce oxidative stress in the skin, which leads to its protective barrier dysfunction or to the immune deregulation. Moreover, it has been shown how the aryl hydrocarbon receptor (AhR) activation appears to be also involved (5, 6, 7).

Air pollution is an aggravating factor in several skin inflammatory pathologies, including acne. Consequently, often dark spots appear in normal skins and acne appears in fatty skins during very high air pollution levels. A positive correlation between air environmental pollution and the generation and evolution of acne exists because a study has established that, the higher the concentration of certain atmospheric pollutants, the greater the number of medical consultations of *acne vulgaris* patients is, and that at higher concentrations of certain atmospheric pollutants, also the sebum secretion and acne skin lesions grows (8, 9).

Furthermore, environmental air pollution cause skin sebum oxidation, which intensifies acne signs. Even more, atmospheric pollution in combination with environmental dust and ultraviolet (UV) radiation originating from solar irradiation results in squalene oxidation, the main sebum component.

The relationship between premature skin aging and air environmental pollution exposure was firstly observed in the epidemiologic study SAILA, carried out on advanced age Caucasic women, wherein a correlation between a greater exposure to particulate matter originating from road traffic and a more advanced skin aging was found. In particular, it was correlated with the appearance of greater quantity and greater depth wrinkles (nasolabial fold) together with an increase in the number of spots in cheeks and forehead. In fact, an association between a distance of 100 m or less to a busy traffic road with a 35 % more forehead spots and 15 % more cheek spots was found (10).

But not only an exposure to particulate matter is associated with an accelerated skin aging. A strong correlation between $NO_2$ and the formation of spots in Caucasic women has also been found (n = 806; SAILA). These results were also confirmed in Chinese women (n + 1,072; Thaizhou longitudinal study cohort). An increase of 10 $\mu g/m^3$ $NO_2$ was associated with 25% more spots in German women (p = 0.003) and a 24 % increase in Chinese women more than 50 years old (11). As additional data, many United Kingdom cities present such $NO_2$ levels that surpass the legal limits, with London overtaking the annual threshold in the first week of 2016, with $NO_2$ concentrations reaching more than 200 $\mu g/m^3$ $NO_2$.

Environmental air pollution causes short-term effects on the skin, for example, dryness increases, reddening and irritation and other long-term effects, like premature aging, collagen degradation in addition to spots and deeper wrinkles appearance.

Current skin treatment and care products are developed to cover a wide spectrum of effects caused by the environmental air pollution, thus, there being some anti-wrinkle products, some are anti-acne, some are moisturising, etc. The application of all these products to alleviate the environmental air pollution effects on the skin implies a significant expenditure and the parallel or simultaneous application of several formulations on the skin, with the associated risk of producing unwanted effects, given that said product combinations have not been previously tested.

### SUMMARY OF THE INVENTION

A first aspect of the invention refers to a composition for treating the air pollution effects on the skin, which

comprises diterpenes, oleuropein, verbascoside, quercetin and hydroxytyrosol.

The invention also refers to a dose of between 0.1 to 1000 milligrams of a composition that comprises diterpenes, oleuropein, verbascoside, quercetin and hydroxytyrosol.

Moreover, the invention refers to a formulation for parenteral, transdermal, oral or topic administration, wherein said formulation contains a composition that comprises diterpenes, oleuropein, verbascoside, quercetin and hydroxytyrosol.

Another aspect of the present invention refers to the cosmetic use of said composition, that comprises diterpenes, oleuropein, verbascoside, quercetin and hydroxytyrosol, to cosmetically alleviate the aesthetic environmental pollution effects on the skin, to prevent premature skin aging associated with the air pollution effects on the skin.

Another additional aspect refers to the composition that comprises diterpenes, oleuropein, verbascoside, quercetin and hydroxytyrosol, for its use as medicament; in particular, for its use in the treatment or prevention of skin affections caused by environmental pollution.

Finally, the present invention refers to a dietetic supplement, or a nutricosmetic, that comprises said composition that comprises diterpenes, oleuropein, verbascoside, quercetin and hydroxytyrosol.

## BRIEF DESCRIPTION OF THE DRAWINGS

**Figure 1.** Graphic that shows the percentage of human keratinocyte oxidation (HaCaT) treated with compositions 1B of the invention, 2B and 3B, at two different concentrations in comparison with the untreated cells.

**Figures 2A, 2B and 2C.** Graphics that show the percentage of human keratinocytes oxidation (HaCaT) *in vitro* incubated with the three formulations of Table 1 (1B of the invention, 2B and 3B) and exposed to type B ultraviolet radiation (UVB), with intensities of 800 and 1200 $J/m^2$ in comparison with untreated and non-irradiated samples.

**Figure 3.** Graphic that shows the relative human keratinocytes IL-8 levels (HaCaT) *in vitro* in comparison with untreated and irradiated cells. Keratinocytes were treated with 10 $\mu$g / ml of different formulations and were exposed to UVB radiation (300 $J/m^2$). Data are expressed as an average $\pm$ SD. The use of different formulations was carried out during and after irradiation and also only after irradiation.

**Figure 4A, 4B and 4C.** Graphics that show the percentage of melanin produced by human melanocytes treated with thee formulations of Table 1 (1B of the invention, 2B and 3B) in comparison with untreated melanocytes.

**Figure 5.** Columns graphic representing the accumulation of ROS in human keratinocytes samples (HaCaT) treated with the 1B composition of the invention (ZeroPollution) at 0.001 % and 0.005 % weight concentration and subjected to 24 hours to urban dust matter (UD) at a 400 $\mu$g/ml concentration and subsequently irradiated with UVA during 25 minutes. Data have been normalised with respect to the control exposed to the same UD amount and UVA radiation.

**Figure 6.** Graphic showing the malondialdehyde (MDA) concentration increase in *ex vivo* skin explants after being exposed to a pollutants mixture and untreated (control bar) and treated with 1B formulation (ZeroPollution bar).

**Figure 7.** Graphic showing the IL1-alpha concentration in *ex vivo* skin explants. First column: without being exposed to the pollutants mix and without treatment; Second column: after being exposed to the pollutants mix and without treatment; and Third column: after being exposed to pollutants mix and with 1 B composition of the invention treatment (ZeroPollution).

**Figure 8.** Graphic showing the AhR expression in *ex vivo* skin explants. First column: without being exposed to the pollutants mix and without treatment; Second column: after being exposed to the pollutants mix and without treatment; and Third column: after being exposed to pollutants mix and with 1B composition of the invention treatment (ZeroPollution).

**Figure 9.** Graphic showing the metallothionein MT-H1 protein expression in *ex vivo* skin explants. First column: without being exposed to the pollutants mix; Second column: after being exposed to the pollutants mix; and Third column: after being exposed to pollutants mix and with 1B composition of the invention treatment (ZeroPollution).

**Figure 10.** Graphic showing the skin hydration measured as a % of the hydration change from the beginning of the

study, T0, at 14, 28, 56 and 84 days of use of the composition of the invention (ZeroPollution, solid black bars) or with placebo (horizontal lines bars).

**Figure 11.** Graphic that shows the transepidermal water loss measured as % hydration change from the beginning of the study, T0, at 14, 28, 56 and 84 days of use of the composition of the invention (ZeroPollution, solid black bars) or with placebo (horizontal lines bars).
of change in

**Figure 12.** Graphic that shows the change in sebum secretion produced in contact with pollution at 14, 28, 56 and 84 days of use of the composition of the invention (ZeroPollution solid black bars and placebo in horizontal lines bars).

**Figure 13.** Graphic that shows the change in skin elasticity at 14, 28, 56 and 84 days of use of the composition of the invention (solid black bars), in comparison with placebo use (horizontal lines bars).

**Figure 14.** Change in skin firmness (measured as R0 absolute values decrease, as representation of the skin firmness increase), at 14, 28, 56 and 84 days of use of the composition of the invention (solid black bars), in comparison with placebo use (horizontal lines bars).

**Figure 15.** Change (in %) of skin luminosity (measured as cheek brightness value) at 14, 28, 56 and 84 days of use of the composition of the invention (solid black bars), in comparison with placebo use (horizontal lines bars).

**Figure 16.** Change (in %) of ITA value measured in a dark spot at 14, 28, 56 and 84 days of use of the composition of the invention (solid black bars), in comparison with placebo use (horizontal lines bars).

**Figure 17.** Change (in %) of wrinkles depth at 14, 28, 56 and 84 days of use of the composition of the invention (solid black bars), in comparison with placebo use (horizontal lines bars).

**Figure 18.** Change (in %) of skin uniformity at 14, 28, 56 and 84 days of use of the composition of the invention (solid black bars), in comparison with placebo use (horizontal lines bars).

**Figure 19.** Change (in %) of the MDA production at 28, 56 and 84 days of use of the composition of the invention (black solid bars), versus the placebo use (horizontal lines bars)

**Figure 20.** Change (in %) in FRAS (Saliva reducing ferric antioxidant) at 28 and 84 days of use of the composition 1B of the invention (solid black bars), versus the placebo use (horizontal line bars).

## DESCRIPTION OF THE INVENTION

A first aspect of the invention provides a composition for treating the air pollution effects on the skin, said composition comprising diterpenes, oleuropein, verbascoside, quercetin and hydroxytyrosol. In a preferred embodiment the composition of the invention comprises carnosic acid. Preferably, the composition comprises carnosic acid as main diterpene.

In one embodiment the composition comprises carnosic acid, oleuropein, verbascoside, quercetin and hydroxytyrosol

A composition as previously described is suitable for the treatment of the air pollution effects on the skin because it causes a combination of effects on it needed to alleviate or counteract all harmful air pollution effects. This is to say, a composition as previously described imparts important and marked antioxidant, anti-inflammatory, anti-aging and whitening effects on human skin, i.e. said composition provides cosmtic effects (anti-aging) as well as pharmaceutical (anti-inflammatory). In this way, the simultaneous application of several skin-care products in order to prevent all air pollution associated affections, such as inflammation, acne increase, chronic dermatitis, psoriasis, etc, can be avoided. The use of a composition like the one previously described can be preventive or to treat existing skin conditions.

Therefore, an embodiment refers to a composition that comprises diterpenes, oleuropein, verbascoside, quercetin and hydroxytyrosol.

In one embodiment, the composition comprises an individual ration of 2.5 to 5 in weight of each of diterpenes, oleuropein, verbascoside and quercetin to hydroxytyrosol, approximately. It is to say, in one embodiment the composition of the invention comprises a diterpenes to hydroxytyrosol ratio of 2.5 to 5, an oleuropein to hydroxytyrosol ratio of 2.5 to 5, a verbascoside to hydroxytyrosol ratio of 2.5 to 5 and a quercetin to hydroxytyrosol ratio of 2.5 to 5.

Preferably, the diterpenes to hydroxytyrosol weight ratio is around 3, the oleuropein to hydroxytyrosol weight ratio is around 3, the verbascoside to hydroxytyrosol weight ratio is around 4.5 and the quercetin to hydroxytyrosol weight ratio is around 2.5.

For the effects of this invention, the terms "approximately" and "around" referred to a numerical value, ratio or percentage indicate that said numeric value, ratio or percentage, can vary $\pm$ 10% of its value. Thus, around 2.5 includes al values comprised between 2.25 and 2.75, i.e., for example 2,25; but also 2.257; 2.31; 2.54, 2.5; 2.62; 2.63; 2.7; 2.75, etc and all values among them.

These weight rations confer the composition a suitable balance among the antioxidant, anti-inflammatory, anti-aging and whitening (or anti- excessive pigmentation) effects so that the prevention and/or treatment of the different environmental air pollution effects is effective and tangible with a single product.

A composition with the aforementioned features is only achievable by the combination of extracts of different plants. Each extract may provide one or several on the active ingredients, or a part of the total content of each active ingredient.

In one embodiment, as quercetin source several extracts may be used, such as, for example, onion, fennel, dill, cranberry or chrysanthemum extracts, but the preferred quercetin source is *Sophora japonica* extract with a minimum quercetin content of 40 % weight. In a preferred embodiment, the composition comprises a *Sophora japonica* extract with a minimum quercetin content of 40 %weight. Preferably, the *Sophora japonica* extract provides a minimum quercetin content of 3.5 % of the total composition weight.

A *Sophora japonica* extract with a minimum quercetin content of 40 %weight is a beneficial ingredient for this composition because it provides a high quercetin concentration in relation with its manufacturing cost and weight and allows us to obtain satisfactory concentrations of the remaining active ingredients.

Quercetin is a flavonoid belonging to the flavonol family. It can be found mainly in onions, grapes, berries, cherries, broccoli and citrus fruits. Quercetin is a potent anti-oxidant with high free-radical scavenging activity besides having a good anti-inflammatory activity. Another interesting property of quercetin is that there are studies in which it has been shown that it reduces histamine release capacity (12). Several studies have proven that certain polluting agents like diesel particulates increase histamine levels, thus increasing the allergic reactions in people living in highly polluted areas.

In one embodiment, the diterpenes source may be one or a combination of plant extracts. Diterpenes have a pronounced anti-inflammatory effect, which is very beneficial for a composition for alleviating the effects of environmental air pollution on the skin.

Carnosic acid is a diterpene found in numerous easily available plants and therefore is a suitable diterpene to be used in this composition due to its suitable cost and abundance. In one embodiment, the carnosic acid source is an extract or a combination of extracts of rosemary, sage, oregano, Melissa or thyme, for example.

Preferably, the composition comprises a rosemary extract with a minimum diterpenes content of 15 % weight. Said extract provides a high diterpenes concentration in relation to its manufacturing cost and weight and allows obtaining satisfactory concentration of the remaining active ingredients. Preferably said rosemary extract provides a minimum diterpenes content of 4.5 % of the total composition weight of the invention.

Carnosic acid also has a potent anti-oxidant activity, an important anti-inflammatory activity, due to its ability to eliminate interleukins and to block or inhibit nitrous oxide release and the tyrosine-kinase pathway. Carnosic acid has also anti-adipogenic properties and prevents premature skin aging by the inhibition of cutaneous lipid oxidation or by biophylactic mechanisms.

In one embodiment, the oleuropein source is an extract or combination of plant extracts, for example, olive leaves or fruits extracts or argan extracts or ash tree extracts. Preferably, the composition comprises an olive (leaf or fruit) extract with a minimum content of 16 % weight in oleuropein. Preferably said olive extract provides a minimum oleuropein content of 4.5 % weight of the total composition weight of the invention.

An olive extract with a minimum oleuropein content of 16 % weight is a very suitable oleuropein source because olive extracts are easy to manufacture at a suitable cost and is a very abundant tree in the Mediterranean basin. In addition, an oleuropein content of at least 16 % weight is enough to assure a suitable bioactivity when combined with other extracts.

Oleuropein exhibits an important anti-oxidant activity implemented through several mechanisms. Mainly, oleuropein enhances free-radical stability by hydrogen bridge formation between the hydroxyl group free oxygen and its phenoxyl radicals. Likewise, oleuropein exhibits anti-inflammatory effects through lipoxygenase activity, leukotriene B4 production, pro-inflammatory cytokine biosynthesis inhibition or inflammation parameters modulation.

In one embodiment, the hydroxytyrosol source is an extract or a combination of plant extracts, for example, olive leaves or fruits extracts or argan extracts or ash tree extracts. Preferably, the composition comprises an olive (leaf or fruit) extract with a minimum content of 5 % weight hydroxytyrosol. Preferably said olive extract provides a minimum hydroxytyrosol content of 1.5 % weight of the total composition weight of the invention.

An olive extract with a minimum hydroxytyrosol content of 5 % weight is a very suitable hydroxytyrosol source

because olive extracts are easy to manufacture at a suitable cost and is a very abundant tree in the Mediterranean basin. In addition, a hydroxytyrosol content of at least 5 % weight is enough to assure a suitable bioactivity when combined with other extracts.

The great antioxidant activity of hydroxytyrosol is attributed to the o-dihydroxyphenyl molecular fragment thanks to its ability to inhibit the fee-radical effects and to its $Fe^{+3}$ ion reducing power. The antioxidant properties of o-diphenols are associated with their capacity to form intramolecular hydrogen bridges between the hydroxyl group and the phenoxyl radical; thus, the catechol group prevents the chain propagation by donating a hydrogen radical to the alkyl peroxide radicals formed in the initial step of lipid oxidation.

Inflammation and its consequences play a crucial role in skin affections. It has been proven that hydroxytyrosol inhibits both the production of proinflammatory leukotriene molecules and the arachidonate lipoxygenase activity. Recently it has been published that an extract with 20 % weight hydroxytyrosol inhibits inflammatory swelling and hyperalgesia and supresses proinflammatory cytokines in an inflammation rat model.

In one embodiment, the verbascoside source is an extract or a combination of plant extracts, for example, olive leaves or fruits extracts, *Buddleja globosa, Lippia citriodora, Plantago lanceolata, Cistanche tubulosa, Rehmannia glutinosa, etc.* Preferably, the composition comprises a *Lippia Citriodora* extract with a minimum verbascoside content of 25 % weight. Preferably said olive extract provides a minimum verbascoside content of 6.5 % weight of the total composition weight of the invention.

A *Lippia Citriodora* extract with a minimum verbascoside content of 25 % weight is a very suitable verbascoside source because extracts form this plant are easy to manufacture at a suitable cost due to its abundance in the Mediterranean basin. Moreover, a minimum verbascoside content of 25% weight is enough to assure an adequate bioactivity when this extract is combined with other extracts.

Verbascoside, also known as acetoxide, exhibits antioxidant activity, anti-inflammatory activity, whitening activity, photoprotective activity and chelating activity. The antioxidant activity has been proven in numerous studies. The anti-inflammatory activity has been proven in an *in-vitro* study on human keratinocytes cultures, in which verbascoside was able to reduce the release of proinflammatory chemokines in a dose-dependent response (13). Verbascoside inhibits tyrosinase activity and melanin production in melanoma B16 cells, according to a study by Young *et al.* (13, 14).

In one embodiment, the composition comprises between 25 % and 40 % weight of a rosemary extract providing a minimum diterpenes content of 4.5 % weight of the total composition weight, between 20 % to 40 % weight of a olive leaves or fruits extract providing a minimum oleuropein content of 4.5 % weight of the total composition weight and a minimum hydroxytyrosol content of 1.5 % of the total composition weight, between 20 % and 35 % weight of a *Lippia Citriodora* extract providing a minimum verbascoside content of 6.5 % of the total composition weight and between 5 % and 25 % weight of a *Sophora Japonica* extract providing a minimum quercetin content of 3.5 % of total composition weight.

Preferably, the composition comprises around 32 % weight of a rosemary extract providing a minimum diterpenes content of 4.5 % weight of the total composition weight, around 30 % weight of a olive leaves or fruits extract providing a minimum oleuropein content of 4.5 % weight of the total composition weight and a minimum hydroxytyrosol content of 1.5 % of the total composition weight, around 28 % weight of a *Lippia Citriodora* extract providing a minimum verbascoside content of 6.5 % of the total composition weight and around 10 % weight of a *Sophora Japonica* extract providing a minimum quercetin content of 3.5 % of total composition weight.

In one embodiment, the composition comprises a minimum diterpenes content of 4.5 % (weight/weight), a minimum oleuropein content of 4.5 % (weight/weight), a minimum hydroxytyrosol content of 1.5 % (weight/weight), a minimum verbascoside content of 6.5 % (weight/weight) and a minimum quercetin content of 3.5 % (weight/weight), in the total composition weight.

More preferably, the composition comprises around 32 % weight of an rosemary extract with a minimum diterpenes content of 15 % weight, around 30 % weight of a olive leaves or fruits extract with a minimum oleuropein content of 16 % weight and a minimum hydroxytyrosol content of 5 % weight, around 28 % weight of a *Lippia Citriodora* extract with a minimum verbascoside content of 25 % weight and around 10 % weight of a *Sophora Japonica* extract with a minimum quercetin content of 40 % weight.

The compositions described in the present document exhibit a suitable balance among the antioxidant, anti-inflammatory and whitening effects on the skin, as demonstrated by our studies of antioxidant activity in keratinocytes under exposure and in absence of UV radiation, of anti-inflammatory activity in keratinocytes under UV radiation exposure and whitening activity in melanocytes. It is very convenient to have antioxidant, anti-inflammatory and whitening functions balanced in a single composition, so that it is feasible to alleviate the multiple environmental air pollution effects on the skin by applying a single product, which has been adequately tested. In contrast, if we try to treat the multiple environmental air pollution effects on the skin by applying diverse products for each effect, the risk of using incompatible products increases, given that their combined use has not been previously tested, and it is possible that the treatment used causes a worse effect than the initial problem. Furthermore, the fact of using a single product implies a lower cost of daily skin care.

The composition of the present invention may be parenterally, transdermally, orally or topically administered.

One embodiment refers to the cosmetic use of the composition of the present invention.

Therefore, one embodiment refers to the cosmetic use of the composition of the present invention to cosmetically alleviate the aesthetic environmental pollution effects on the skin.

Another embodiment refers to the composition of the present invention for its use as cosmetic with anti-oxidant, anti-inflammatory aesthetic effects, taking this effect not as a therapeutic treatment but as merely aesthetic, antiaging and whitening treatment on the skin.

Preferably, said use comprises administering an oral dose between 0.1 g and 1 g (1000 mg) daily of the composition of the invention, described in the present document. More preferably, the oral dose is 250 mg daily.

Thus, one embodiment refers to the use of the composition of the invention, described in the present document, for the manufacture of a cosmetic antioxidant, anti-inflammatory product, taking this effect not as a therapeutic treatment but merely aesthetic, antiaging and whitening on the skin.

One embodiment refers to the composition of the invention, described in the present document, for its use as a medicament.

Particularly, one embodiment refers to the composition of the invention, described in the present document, for its use in the treatment or prevention of skin affections caused by environmental pollution. Preferably, said use comprises administering an oral daily dose of between 0.1 g and 1 g (1000 mg) of the composition of the invention, described in the present document. More preferably, the oral dose is 250 mg daily.

Preferably, said affections are selected form the group consisting of inflammation, acne, chronic dermatitis and psoriasis.

Another embodiment refers to the use of the composition of the invention, described in the present document, for the manufacture of a medicament for its use in the treatment or prevention of skin affections caused by environmental pollution.

Another embodiment refers to a method of treatment or prevention of skin affections caused by environmental pollution, that comprises administering an individual an effective amount of the composition of the invention, described in the present document.

It is understood that "effective amount", to the effects of the present invention, as such that provides a therapeutic effect without causing unacceptable toxic effects in the patient. The effective amount or dose of the composition depends on, for example, the treated affection, age, weight and clinical condition of the treated patient, the administration form, the patient clinical history, the severity of the treated affection and the potency of the administered composition. Preferably said effective amount or dose is an oral daily dose of between 0.1 g and 1 g (1000 mg) of the composition of the invention, described in this document.

The previously described composition may be administered in a dose between 0.1 g and 1 g daily. This dose is considered safe according to the toxicity studies carried out and shown in example 4.

Preferably, the daily dose is 250 mg. This dose produces significant and noticeable effects in the alleviation of the environmental air pollution effects on the skin compared to pharmaceutically or cosmetically non-treated models or people.

Another second aspect of the invention provides a composition for its use in the prevention of premature skin aging associated with the environmental air pollution effects on the skin, said composition comprising diterpenes, oleuropein, verbascoside, quercetin and hydroxytyrosol. Said cosmetic use provides a reduction of the pollution effects, preventing, therefore, the premature skin aging.

The features of the composition, in general, and in particular in what it refers to its composition, components ratios and its sources, of the first aspect of the invention are equally applicable to the composition of the second aspect of the invention.

In a more particular embodiment, the administration of the compositions of the present invention is selected among the formulations that contain it intended for oral, topical, transdermal or parenteral administration.

Preferably, the compositions of the present invention may be administered orally containing one or more physiologically compatible vehicles or excipients, in solid or liquid form. Thus, another aspect of the invention refers to a formulation that comprises the composition of the invention, as described in the present document, and one or more physiologically compatible vehicles or excipients.

One embodiment refers to a pharmaceutical formulation that comprises the composition of the invention, as described in the present document, and one or more pharmaceutically acceptable vehicles or excipients.

Another embodiment refers to a cosmetic formulation that comprises the composition of the invention, as described in the present document, and one or more cosmetically acceptable vehicles or excipients.

In a particular embodiment, said cosmetic formulation is a dietetic supplement. An embodiment of the invention refers, therefore, to a dietetic supplement that comprises the composition of the invention, as described in the present document. Another embodiment refers to the use of said dietetic supplement to cosmetically alleviate the environmental pollution aesthetic effects on the skin.

In another particular embodiment, said cosmetic formulation is a nutricosmetic. An embodiment of the invention refers, therefore, to a nutricosmetic that comprises the composition of the invention, as described in the present document.

Another embodiment refers to the use of said nutricosmetic to cosmetically alleviate the environmental pollution aesthetic effects on the skin.

The term nutricosmetic refers to, to the effects of the present invention, to a food, of part of a food, that provides cosmetic benefits when ingested.

Thus, one embodiment refers to the use of a cosmetic formulation, described in the present document, for the manufacture of a cosmetic antioxidant, anti-inflammatory, antiaging and whitening product.

Another embodiment refers to the use of a pharmaceutical formulation, described in the present document, for the manufacture of a medicament for the treatment or prevention of skin affections caused by environmental pollution.

Particularly, one embodiment refers to the use of a pharmaceutical formulation, described in the present document, for use in the treatment or prevention of skin affections caused by environmental pollution.

Another embodiment refers to a method of treatment or prevention of skin affections caused by environmental pollution, that comprises administering to an individual the cosmetic or pharmaceutical formulation of the invention, described in the present document.

Said formulations may contain conventional components such as physiologically acceptable excipients, vehicles or binding agents, fillers, lubricants and moisturising agents. The formulations may adopt any suitable form and can be administered orally, nasally, topically or parenterally, such as creams, balms, vaporisers, pills, dragees, capsules, lozenges, oily or aqueous solutions, suspensions, emulsions or dry powder form apt for pre-use reconstitution in water or other liquid medium, for immediate or controlled release.

To manufacture said formulations, conventional techniques for the manufacture of pharmaceutical or cosmetic formulations may be used. When the vehicle serves as solvent, it can be solid, semi-solid or liquid and act as excipient or medium for said active compound. The composition of interest can be absorbed in a granular solid medium. Some examples of suitable vehicle are water, saline solutions, alcohols, polyethylenglycols, polyhydroxyetoxylated castor oil, peanut oil, olive oil, lactose, terra alba, saccharose, cyclodextrins, amylose, magnesium stearate, talcum, gelatine, agar, pectin, acacia, stearic acid, alkyl-cellulose ethers, silicon acid, fatty acids, fatty acid amines, fatty acid monoglycerides and diglycerides, pentaerytrol fatty esters, polyethylene, hydroxymethylcellulose, microcrystalline cellulose and polyvinylpyrrolidone.

In order to prepare solid formulations such as pills, the compound of interest is mixed in a formulation with other conventional ingredients such as talcum, magnesium stearate, dicalcium phosphate, aluminium and magnesium silicate, starch, lactose, acacia, methylcellulose, microcrystalline cellulose and functionally similar materials such as pharmaceutical vehicles and diluents.

The liquid oral forms may also contain certain additives such as sweeteners, flavourings, preservatives and emulsifying agents. The non-aqueous liquid compositions for oral administration may also be formulated containing edible oils, for example. Such liquid compositions may be encapsulated in a suitable way, for example, in gelatine capsules in unitary dose amounts.

The capsules may be prepared mixing the compound of interest with a pharmaceutically inert solvent and filling the mixture in a hard gelatine of suitable size. Soft capsules are prepared with encapsulating machines of suspensions of the compounds of interest in a vegetal oil, a light paraffin or an acceptable inert oil. Liquid dosage forms can also be prepared, such as syrups, elixirs, and suspensions. The water soluble forms may be dissolved in an aqueous vehicle together with sugar, flavouring aromas and preservatives to form a syrup. An elixir is prepared using an hydroalcoholic vehicle (ethanol for example) with suitable sweeteners such as sugar or saccharine, together with flavouring aromatic agents. The suspensions may be prepared with an aqueous vehicle and the aid of a suspension agent such as acacia, tragacanth, methyl cellulose and the like.

In one embodiment, said composition for cosmetic use according to the invention is administered orally in a dose between 0.1 g and 1000 mg daily. Preferably, the dose is 250 mg daily. In particular, said composition for cosmetic use according to the invention is administered in a dietetic supplement or as nutricosmetic that comprises a dose of between 0.1 to 1000 mg to be administered orally.

In one embodiment, said dietetic supplement comprises additional excipients, preferably water, saline solutions, alcohols, polyethylenglycols, polyhydroxyetoxylated castor oil, peanut oil, olive oil, lactose, terra alba, saccharose, cyclodextrins, amylose, magnesium stearate, talcum, gelatine, agar, pectin, acacia, stearic acid, alkyl-cellulose ethers, silicon acid, fatty acids, fatty acid amines, fatty acid monoglycerides and diglycerides, pentaerytrol fatty esters, polyethylene, hydroxymethylcellulose, microcrystalline cellulose and polyvinylpyrrolidone.

In another additional embodiment said dietetic supplement is a capsule. Preferably a hard gelatine capsule.

The compositions of the present invention may be administered parenterally in combination with injectable liquid vehicles, such as water and suitable alcohols. The conventional pharmaceutical adjuvants to injection, such as stabilising agents, solubilising agents and buffers may also be included in such injectable formulations. The composition of the present invention may be intramuscularly, intraperitoneally or intravenously administered.

Likewise, the composition of the present invention may be topically administered. The topical administration formulations may contain one or more vehicles or excipients physiologically compatible with the skin, in liquid, aerosol,

cream forms, etc.

In order to prepare the formulations of topical application, the composition of the invention is included in a dermatologically accepted vehicle. The composition amount to be administered and the composition amount in the topical use formulation depends on the vehicle, on the individual to whom it is administered, the desired effects and formulation stability. Thu, the skilled person will know how to use the appropriate preparation containing the proper composition amount and will select the formulation amount to administer, depending on the previously indicated conditions.

Preferable, said composition for cosmetic use according to the invention is topically administered in a cosmetic product that comprises 0.05 % to 2 % weight of the composition of the invention. Preferably, from 0.1 % to 1 % the cosmetic product comprises from 0.1 % to 1 % of the composition of the invention, to be topically administered.

**Molecular mechanisms of air pollution effects on the skin**

Atmospheric pollution, in combination with ozone and UV radiation, alter the existing balance between reactive oxygen species (ROS) production and elimination which implies the induction of an inflammatory response. Atmospheric pollution induced chronic stress impacts and alters the skin barrier integrity to such extent that it can generate or worsen certain pathologies (sensibility, irritability, dehydration, pigmentation alteration, dermatitis, acne, etc.) (15).

Ozone acts mainly on the *stratum corneum,* in contrast, particulate matter (PM) contains a series of toxic molecules, such as polycyclic aromatic hydrocarbons (PAHs), which, upon contact with the skin, can penetrate it and harm it in a deeper level, directly acting on the keratinocytes and melanocytes, for example, by binding with the AhRs (aryl hydrocarbon receptors), which activation stimulates the expression of genes involved in skin aging, pigmentation and oxidative stress which causes skin inflammation (15).

In this way, a compound that is able to inhibit the AhR overactivation, which is associated with skin inflammation (itch hypersensibility) and with spots appearance and the worsening of existing conditions is a good strategy to obtain an anti-pollution composition.

In this sense, one of the objectives of the formulation is to be able to inhibit AhRs activation as a consequence of air pollution.

As it has been already mentioned, one of the mechanisms by which the environmental air pollution can damage the skin includes the AhR activation. Environmental air pollution generates free radicals, skin immune-inflammatory responses cause the activation of mechanism dependent of the AhR activation and alter the skin microflora.

Several scientific studies have demonstrated the involvement of the AhR activation in several skin affections. A study about the involvement of the AhR activation in transgenic mice proved that the AhR transcription activation caused inflammatory skin lesions similar to atopic dermatitis. The micro array genetic analysis of the mice skin showed an overexpression of inflammation associated genes. The results of this study prove that the AhR activation might be involved in the development of skin inflammatory diseases.

AhR is a ligand activated cytosolic transcription factor present in various skin cell types which regulate the proliferative cell inflammation and melanogenesis, including keratinocytes, fibroblasts and melanocytes (16, 17). The non-activated AhR is found in a cytosolic protein complex and upon binding with a ligand, the complex dissociates, thus causing the AhR translocation to the nucleus, where it stimulates the expression of genes which contain the xenobiotic response element (XRE). The genes that contain the xenobiotic response element (XRE) include cytochrome detoxification enzymes P450 (CYP) (13). The AhR activation plays a mediating role between the biochemical and toxic effects of the environmental air pollutants that act as ligands, such as dioxins, ozone and polycyclic aromatic hydrocarbons (PAHs), thus leading to influence cellular biologic processes such as growth and differentiation and it has been recently proven that they can elicit melanocyte proliferation and therefore darkening of mice skin (18).

The polycyclic aromatic hydrocarbon 2,3,7,8-tetrachlorodibenzene-p-dioxin (TCDD) has several pathologic effects in humans through the AhR activation. The primary mechanism subjacent to the TCDD induced genetic expression changes is the activation of the transcription cycle which involves the AhR and the AhR translocator (Arnt). AhR and Arnt are transcription factors involved in the development regulation, hypoxia signalling and circadian rhythms, and belong to the protein family that is found in the cytoplasm in an inactive complex with auxiliary proteins.

Once bound to the TCDD, the AhR dissociates from some of the inactive complex proteins and translocates to the nucleus where it dimerises with Arnt. The heterodimer AhR/Arnt activates the transcription of xenobiotic metabolising genes, some of which codify proteins involved in growth control, cytokines, nuclear transcription and extracellular matrix proteolysis regulators (19).

The AhR cycle is activated by TCDD and by trans-retinoic acid (all-trans retinoic acid), which increase the MMP-1 expression in human normal keratinocytes, which suggests that MMP induction is a common mechanism subjacent to the TCDD induced pathologies (20).

It has been proven that AhR is a melanogenesis regulator. Several studies have examined the AhR role, and an example is a study in which the melanocytes of human donors were exposed to TCDD and subsequently the tyrosinase

activity and the melanin content was measured *in-vitro.* After 5 days of exposure to TCDD, the melanocytes showed an increased tyrosinase activity level and a three-fold increase in intracellular melanin. A similar experiment carried out in FM55 human melanoma cells proved that TCDD significantly increases the melanin content, thus confirming that the melanogenic cycle was overregulated by AhR activation (21).

AhR has also been identified as an ozone sensor. Ozone induces the AhR nuclear translocation and increases the AhR expression by mRNA. Furthermore, ozone significatively increases the protein expression and CYP1 isoform mRNA. CYP1 enzymes metabolise compounds which are more toxic than the original respective compounds and activate many xenobiotic procarcinogenic compounds (22).

With the aim to analyse the AhR involvement in tobacco smoke induced skin ageing (also considered a polluting agent), human primary fibroblasts were exposed to a hexane soluble tobacco smoke extract. This extract significantly increased MMP-1 induction in a human fibroblast culture, in parallel with a P1B1 cytochrome overexpression. A reduction in the AhR amount supressed the elevated Ahr dependent gene transcription, CYP1A1/CYP1B1, induced by the extract. The MMP-1 and CYP1B1 induction was supressed by the AhR 3-metoxy-4-nitroflavone and α-naphthoflavone cycle inhibitors. These studies suggest that the hexane soluble tobacco smoke extract induces MMP-1 overexpression in human skin fibroblasts through the AhR cycle activation. Therefore, the AhR cycle might be pathogenically involved in skin aging cause by external factors (23).

Whereas the correlation between the air pollution and the predominance and severity of atopic dermatitis is well known, the subjacent mechanism is not that well known. Currently, atopic dermatitis symptoms are treated with steroidal compounds. However, in many cases, itchiness persists. One of the reasons would be the AhR activation in response to air pollutants, such as traffic derived particulate matter. The activated AhR induces the expression of the artemin neurotrophic factor in keratinocytes, which includes the epidermis hyper-enervation, thus resulting in itchiness hypersensibility. The subsequent scratching implies an alteration of the skin barrier and an increased antigen penetration, which implies sensibilisation and favours the predisposition to other allergic diseases (24).

## EXAMPLES

The examples subsequently described have illustrative character and do not intend to limit the scope of the present invention.

### EXAMPLE 1. Studies of the anti-oxidant, anti-inflammatory and whitening effects of the extracts composition with high flavonoids content.

**TABLE 1.** Composition of the three formulations examined in example 1.

| **Formula 1B** | **mg flavonois/ 100 mg product** |
|---|---|
| *Sophora japonica* extract (10% weight) | 9,4 |
| *Lippia citriodora* extract (28% weight) | 8,4 |
| Rosemary leaves extract (32% weight) | 4,8 |
| Olive leaves extract (30% en weight) | 8,36 |
| **Total Flavonoids** | 30,96 |
| **Formula 2B** | **mg flavonoids/ 100 mg product** |
| *Sophora japonica* extract (10 % weight) | 9,4 |
| *Lippia citriodora* extract (57% weight) | 16,8 |
| Rosemary leaves extract (32 % weight) | 4,8 |
| **Total Flavonoids** | 31 |
| **Formula 3B** | **mg flavonoids/ 100 mg product** |
| *Sophora japonica* extract (10 % weight) | 9,4 |
| Rosemary leaves extract (31 % weight) | 4,8 |
| Olive leaves extract (59% weight) | 16,776 |

(continued)

| Formula 3B | mg flavonoids/ 100 mg product |
|---|---|
| Total Flavonoids | 30,976 |

Preliminary assays to determine the alleviating activity of different compositions on the air pollution effects on the skin have been carried out, bearing in mind that the oxidative and inflammatory processes are very important in the damage generation that pollution causes on the skin.

Moreover, studies about several formulations' capacity to inhibit melanin production have been done, given that air pollution increases the skin pigmentation and the appearance of spots.

Table 1 shows the components and their concentration in each composition examined in the preliminary studies. Table 1 summarises the composition of the three tested formulations and the flavonoids mg per 100 mg of product or final composition that each extract provides to the formulation is indicated.

The total flavonoids contribution of each formulation is the same, around 31 mg per 100 mg of composition or final product. All formulations contain the same amount of *Sophora japonica* and rosemary extracts (always providing the same flavonoids content).

In the study, *in vitro* human keratinocytes cultures were exposed to each of the three formulations, using two different concentrations of each of them and the basal oxidation was measured. Data are expressed as arithmetic averages ± standard deviation (SD). The statistical significance levels are: * ($p< 0.05$), ** ($p<0.01$), *** ($p<0.001$) and **** ($p<0.0001$) in comparison with the control culture that was not treated with any composition.

As it can be seen in Figure 1, formula 1B as well as formula 2B reduced in a significant way the percentage of keratinocytes oxidation (HaCaT) in comparison with the untreated cells (black bar). Contrarily, formula 3B was not able to reduce the cellular basal oxidation at any of the two studied concentrations.

When we compare the formulations among them there are no significant differences in the antioxidant effect of formula 1B and 2B, but there are with respect to formula 3B, at both concentrations.

In another test, which results are shown in Figures 2A, 2B and 2C, the antioxidant activity of the three formulations in Table 1 was measured on *in vitro* cultured human keratinocytes and exposed to type B ultraviolet radiation (UVB), at intensities of 800 and 1200 J/m$^2$.

Data are expressed as arithmetic averages ± standard deviation (SD). The statistical significance levels are: * ($p< 0.05$), ** ($p<0.01$), *** ($p<0.001$) and **** ($p<0.0001$) in comparison with the control culture that received the same radiation intensity and that was not treated with any composition. Table 2 shows the oxidation inhibition percentage in comparison with its respective control.

All tested formulations significantly inhibited the oxidation caused by UVB radiation. However, formulation 1B at a 200 μg/mL concentration was the one that inhibited, almost completely, the UVB induced oxidation, in a percentage significantly greater than formulations 2B and 3B.

**TABLE 2.** Oxidation inhibition percentage in comparison with its respective control.

| Composition | UVB dose (J/m$^2$) | Treatment formulation concentration (μg/mL) | |
|---|---|---|---|
| | | 100 | 200 |
| 1B | 800 | 75.8 | 103.4 |
| | 1200 | 78.1 | 97.5 |
| 2B | 800 | 55.3 | 63.4 |
| | 1200 | 52.8 | 69.9 |
| 3B | 800 | 41.0 | 56.8 |
| | 1200 | 43.3 | 49.9 |

In Figure 3 the results of the study of anti-inflammatory activity of the three formulations in Table 1 are shown. In this test the amount of IL-8 (interleukin 8) produced by human keratinocytes exposed to 300 J/m$^2$ intensity UVB radiation and treated with the formulations of Table 1 at a 10 μg/mL concentration was measured.

Data are expressed as arithmetic averages ± standard deviation (SD). The statistical significance levels are: * ($p< 0.05$), ** ($p<0.01$), *** ($p<0.001$) and **** ($p<0.0001$) in comparison with the control culture that received the same radiation intensity and that was not treated with any composition.

Interleukin 8 is a cytokine produced by keratinocytes and other cells and is associated with inflammatory processes.

In Figure 3 it can be seen that formula 1B as well as formula 2B significantly reduced the inflammation percentage (IL-8 alpha expression) in keratinocytes (HaCaT) in comparison with untreated cells (black bar) when these were incubated during and after irradiation and also when these were incubated only after radiation.

Contrarily, formulation 3B was able to significantly inhibit cytokine IL-8 alpha production only when the keratinocytes were incubated with the formulation after the radiation.

Figures 4A, 4B and 4C show the results of the whitening activity studies of the formulations in Table 1. The graphics represent the melanin percentage produced by melanocytes treated with the formulations in Table 1 in comparison with untreated melanocytes, at the same culture conditions.

Data are expressed as arithmetic averages ± standard deviation (SD). The statistical significance levels are: * ($p< 0.05$), ** ($p<0.01$), *** ($p<0.001$) and **** ($p<0.0001$) in comparison with the melanocytes control culture that was not treated with any composition.

Formulation 2B as well as formulation 3B at the tested concentrations were not able to inhibit the melanin production in a statistically significant amount. Only formulation 1B exhibited such capacity and thus it meets an important requirement in order to obtain a composition for its use in the treatment of the air pollution effects on the skin, given that one of the described effects is a pigmentation increase in people exposed to chronic air pollution.

Flavonoid content determination of the extracts

**HPLC analysis of the *Lippia citriodora* extracts and olive extracts phenolic compounds**

- Standard calibration curve preparation

The calibration curves are prepared with different concentrations of oleuropein, hydroxytyrosol and verbascoside standards. The purity assigned to each standard is the one appearing in the analysis certificate of the standard.

- Dry sample preparation

The samples are dissolved in dimethylsulphoxide (DMSO) to a 5 mg/ml concentration. The resulting solution is filtered with a 0.45 μm nylon membrane.

- HPLC method

Column: LiCrospher 100-C18 (250x4.0 mm i.d.) with an average particle size of 5 μm. Mobile phase flow: 1 ml/min. Temperature: 30 °C. Injection volume: 20 μl.

- Mobile phase: Solvent A: 2.5 % weight acetic acid.

Solvent B: acetonitrile

Gradient:

| Time (min) | Solvent A | Solvent B |
|---|---|---|
| 0 | 9 | 5 |
| 20 | 7 | 25 |
| 40 | 5 | 50 |
| 50 | 2 | 80 |
| 60 | 9 | 5 |

The change in absorbance is monitored at 280 and 330 nm.

Oleuropein, hydroxytyrosol and verbascoside are identified by retention time comparison with the corresponding standard. The concentration is calculated by interpolation in the calibration curve.

**HPLC analysis of the rosemary extract phenolic compounds. Diterpenes.**

- Carnosic acid (CA) standard preparation
  The standard is dissolved to 0.2 mg/ml in DMSO. The resulting solution is filtered with a 0.45 μm nylon membrane.

- Dry sample preparation
  The samples are dissolved in DMSO at a 1 mg/ml concentration. The resulting solution is filtered with a 0.45 μm nylon membrane.

- HPLC method
  Column: LiCrospher 100-C18 (250x4.0 mm i.d.) with an average particle size of 5 μm. Mobile phase flow: 1 ml/min. Temperature: 30 °C. Injection volume: 20 μl.

- Mobile phase. 500 ml water, 935 ml acetonitrile, 2,5 ml phosphoric acid, 0,16 g dihydrate EDTA disodium salt. The change in absorbance is monitored at 230 with a UV-Vis DAD detector.

- Calculations. Carnosic acid (CA) and carnosol (COL) were identified by retention time comparison with their corresponding standards.

% carnosic acid: (AreaCAsample/AreaCAStd x (Wt Std/Wt sample) x CA standard purity

Where:

AreaCAsample: sample CA peak area
AreaCAStd: standard CA peak area
Wt sample: sample weight in mg
Wt Std: CA standard weight in mg

% carnosol: (AreaCOLsample/AreaCOLStd x (Wt Std/Wt sample) x COL standard purity x 0.9

Where:

AreaCOLsample: sample COL peak area
AreaCOLStd: standard COL peak area
Wt sample: sample weight in mg
Wt Std: COL standard weight in mg

**HPLC analysis of the *Sophora japonica* extract phenolic compounds. Quercetin.**

- Quercetin standard preparation
  The standard is dissolved to 0.2 mg/ml in DMSO. The resulting solution is filtered with a 0.45 μm nylon membrane.

- Dry sample preparation
  The samples are dissolved in DMSO at a 1 mg/ml concentration. The resulting solution is filtered with a 0.45 μm nylon membrane.

- HPLC method
  Column: LiCrospher 100-RP18 (250x4.0 mm i.d.) with an average particle size of 5 μm. Mobile phase flow: 1,3 ml/min. Temperature: 50 °C. Injection volume: 20 μl.

- Mobile phase: Methanol: acetonitrile : acetic acid : water in proportions 10 : 10 : 5 : 75. Isocratic method.
  The change in absorbance is monitored at 259 with a UV-Vis DAD detector.
  Quercetin was identified by retention time comparison with its corresponding standard. Its concentration is calculated by interpolation in the calibration curve.

**EXAMPLE 2. In vitro study of the antioxidant and cell protective activity of the formulation 1B against the UV radiation and urban particulate matter effects on human keratinocytes.**

Living organisms are exposed to air pollutants and these have important effects on human skin. Ait pollutants are solid, liquids, gases and particulate matter (PM). These are directly absorbed by the skin until they reach the subcutaneous tissues or through the hair follicles or the sweat/sebaceous cells. The rapid urbanisation and the huge energetic

consumption growth worldwide expose humans to growing amounts of environmental air pollution. The skin, which makes up the largest and the most external organ of the body, acts as a physical, chemical and immunologic barrier against these environmental factors.

Air pollutants exert a harmful effect on the skin through an increase in oxidative stress which acts against the skin antioxidant defences. Free radicals are generated and oxygenated reactive species which interact with lipid-rich plasmatic membranes to initiate a lipid peroxidation reactions cascade. The oxygenated reactive species also trigger proinflammatory mediators release, which results in the neutrophil molecules build up and other phagocytic cells which in turn generated further free radicals, which produces a vicious circle. Moreover, air pollutants produce severe alterations of the normal lipid functions, ARN and/or human skin proteins by oxidative damage, causing extrinsic skin aging, skin allergic or inflammatory conditions, such as contact dermatitis, psoriasis, acne and skin cancer. In the same way, human skin is repeatedly exposed to UV radiation which influences the function and survival of many cell types and is considered the main factor causing skin aging. Acute human skin UV radiation exposure causes sunburn, altered pigmentation, inflammation, immunity suppression and skin connective tissue damage.

Recent studies have proven that sunlight and air pollution might synergistically cause skin stress, accelerate oxidative stress (increased reactive oxygen species production, peroxidation and squalene peroxidation), increase the vitamin E depletion from the skin *stratum corneum,* increase the inflammatory response and even increase the skin cancer risk. Recent studies show that A type UV radiation (UVA) combined with other air pollutants (including tobacco smoke) significantly increases the risk of skin cancer.

It is also highlighted that air pollutants aggravate the damage caused by light and that there is a need of specific skin care strategies in areas with a lot of environmental air pollution.

In this example, human keratinocytes cultures were exposed to urban dust matter and UVA radiation in order to investigate the cellular response to the induced oxidative stress and to verify the formulation 1B potential to counteract the induced damage.

Live cells were counted in a Bürker chamber under microscope. The HaCaT cells were cultured overnight in a 96 well plate with culture medium. After 24 hours, the culture medium was withdrawn and replaced with a new culture medium supplemented with 400 $\mu$g/ml of urban dust matter (UD) and formulation 1B in amounts of 0.005 % and 0.001 % weight.

The reactive oxygen species build up (%ROS) was evaluated in a biological replica, with 8 experimental replicas (in the composition of the inventio as well as in the controls), after being exposed to urban dust matter. After 24 hours of incubation, the culture medium in all wells was replaced with a PBS mixture and a ROS detection master. Subsequently, the samples were irradiated with UVA during 25 minutes at a 6.9 J/cm$^2$ intensity. The non-irradiated controls were kept in darkness all this time. After two hours of the ROS detection mixture addition, the samples ROS concentration was measured by spectrometry, including two control wells without cells, to obtain a blank control. The accumulated intracellular ROS react with a fluorogenic sensor located in the cytoplasm, resulting in a fluorometric product in proportional amounts to the present ROS amount. The fluorometric quantification was measured with a 490 nm wavelength excitation and a 525 nm wavelength emission.

In parallel, a cellular viability analysis was carried out by metabolic reduction of 3-(4,5-dimethyltiazol-2-yl)-2,5-diphenyltetrazol bromide (MTT) in order to assess the cellular mortality induced by the urban dust matter and the UVA radiation, in order to normalise the ROS measured levels to the live cells amount in every specific conditions. In the MTT assays the protocol no. 17 of the European Centre for the Alternative Methods Validation (ECVAM) was followed.

In the cell viability assays, the results show that the formulation 1B use during 24 hours exhibits significative cytotoxicity in HaCaT cells at concentrations equal or above 0.01 % weight, whereas no significative effects were observed at concentrations equal or less than 0.0025 % weight, in comparison with the control sample. According to the results, the 0.001 % and 0.005 % weight concentrations were selected for further studies.

The results from this study indicate that the urban dust matter together with UVA radiation increased the oxidative stress in 13.2 $\pm$ 0.8 times as much, in comparison with the untreated control sample. The samples treated with formulation 1B (ZeroPollution) at 0.001 % and 0.005 % weight concentration significantly reduced the ROS levels in 25.4$\pm$7.2 % and 29.3$\pm$6.2 %, respectively. When removing the basal oxidative stress levels to all samples, the results show that the formulation 1B use at 0.001 % and 0.005 % weight concentrations protects the incubated cells for the urban dust matter and UVA radiation oxidative effects, reducing the oxidative stress in 27.3 $\pm$ 7.8 % and 31.5 $\pm$6.7 %, respectively, in comparison with the control sample exposed to said environmental factors. This fact is reflected in Figure 5.

The bar graphic in Figure 5 represents the ROS build up in the human keratinocytes samples (HaCaT) treated with formulation 1B (ZeroPollution) at 0.001 % and 0.005 % weight concentrations and subject to 24 hours exposure to urban dust matter (UD) at a 400 $\mu$g/ml concentration and subsequently irradiated with UVA during 25 minutes. Data have been normalised with respect to the control exposed to the same UD amount and UVA radiation.

*Represents the statistical significance with a p value < 0.05. ** Represents the statistical significance with a p value < 0.01. *** Represents the statistical significance with a p value < 0.001. **** Represents the statistical significance with a p value < 0.0001.

**EXAMPLE 3. *Ex vivo* evaluation of the anti- air pollution effect activity of the formulation 1B (ZeroPollution) in live human skin explants.**

The objective of this study is the evaluation of the anti-air pollution activity of the formulation 1B in Table 1 on live human skin explants after being treated with a pollutant mixture. After applying *ex vivo* the formulation 1B (ZeroPollution) and a heavy metals, hydrocarbons and diesel particles solution to the explants, the anti-air pollution activity was evaluated by: malonaldehyde biochemical assay, aryl hydrocarbon (AhR) receptor immunostaining, metallothionein MT-1H immunostaining, interleukin-1-alpha (IL-1$\alpha$) biochemical quantitative assay.

19 12mm average diameter human skin explants were obtained by abdominoplasty in a Caucasian 56 years old woman. The explants were kept alive in a culture medium (BIO-EC explant medium) in suitable atmosphere, temperature and humidity conditions.

On day 0, the explants we located in a 2 ml culture medium with and without the product (formulation 1B) at a 200 $\mu$g/mL concentration. The control samples did not receive any treatment. The culture medium (with and without the product to be tested) was renewed with fresh culture medium the first day with 1 ml per well and the second day with 2 ml per well after pollutant exposure.

On the second day, the explants were located in a special culture plate for skin explants and located in the PolluBox® system. 3 ml of pollutants solution were vaporised (heavy metals, hydrocarbons and diesel particles mixture) during an hour and a half. PolluBox® allows to vaporise pollutants in cloud form to contact only the skin surface and thus replicating the physiologic conditions as similarly as possible.

During the exposure, the control samples (not exposed to pollution) were located in a culture plate with 12 wells with 900 $\mu$l HBBS solution per well. After the exposure, all explants were located anew in the incubator at standard conditions, in 2 ml fresh BEM per well.

On day 0 (D0), the three explants from batch T0 were collected and cut in two parts. One part was located in buffered formalin and the other part was frozen at -80 °C.

On day 3, three explants of each batch were collected and treated in the same way than the day 0 explants, and 2 ml of culture medium of each batch were collected and stored at -80°C for the MDA and IL1$\alpha$ assays.

Histologic processing: after the 24 hours buffered formalin fixation, the samples were dehydrated and impregnated with paraffin with the tissue processing apparatus Leica TP1020. The samples were fixed with the paraffin inclusion station Leica EG1160. Dissections of 5 $\mu$m thickness were carried out with the Minot Leica RM 2125 microtome, and the dissections were mounted in Superfrost® glass histologic plates.

The microscopic observations were carried out with a Leica DMLB or an Olympus BX43 microscope. Images were digitalised with an Olympus DP72 numerical camera with the CellD storing software and the image analysis were performed in batches from 9 to 12 images.

MDA biochemical assay

On day 3, MDA content in the culture medium was measured. 4 explants per batch were analysed.

The MDA assay was carried out with an improved TBARs assay method. Th MDA was detected in HBSS medium (Hank's saline solution) by addition of a TBARs solution (thiobarbituric acid, hydrochloric acid, and trichloroacetic acid) and incubation in a water bath at 80 °C during 15 minutes. The MDA was extracted by liquid/liquid butanol extraction. The MDA in butanol was measured by spectrofluorometry at a 515 nm excitation wavelength and a 550 nm emission wavelength, using a Tecan Infinite M200 Pro micro-plate reader. The MDA concentration is expressed in MDA nmoles / ml culture medium.

AhR immunostaining

AhR immunostaining was performed on paraffined dissections with an anti-AhR monoclonal antibody (Thermo Scientific, ref. MA1-514, clon RPT1), diluted 1:50 in PBS-BSA 0,3 % - Tween 20 0,05 % during one hour at ambient temperature with the Vectastain® RTU Universal VECTOR streptavidine/biotine system and revealed with VIP (Vector laboratories, ref. SK4600), which is a violet peroxidase substrate.

MT-1H immunostaining

MT-1H immunostaining was performed on paraffined dissections with an anti-MT-1H monoclonal antibody (Daki, ref. M0639, clon E9), diluted 1:200 in PBS-BSA 0,3 % - Tween 20 0,05 % during one hour at ambient temperature with the Vectastain® RTU Universal VECTOR streptavidine/biotine system and revealed with VIP (Vector laboratories, ref. SK4600), which is a violet peroxidase substrate.

IL1-α biochemical assay

IL1-α biochemical assay was performed with the human IL1-α ELISA kit (Cayman). According with the instructions of the kit supplier, the culture medium and the IL1-α standard were incubated overnight at 4 °C with a acetylcholinesterase:Fab' (AChE/Fab') conjugated antibody, which selectively binds to IL1-α in wells which contain immobilised IL1-a antibody. After washing the wells, the reaction was revealed with a solution that contained the AChE substrate during 40 minutes. The absorbance was measured at 412 nm with the Tecan M200Pro micro-plate reader and the Magellan 7 software.

Results

**MDA**

The malonaldehyde (MDA) is a product formed in the cellular membrane lipid peroxidation. The free radicals induced by the oxidative stress (UVA, pollutants, heavy metals, pesticides...) degrade the polyunsaturated lipds and generate hydroperoxides, which results in the formation of free radical intermediates and aldehydes, particularly MDA.

MDA is one of various low molecular weight end products formed by the decomposition of certain primary and secondary products from lipid peroxidation (25). As it can be seen in Figure 6, on day 3, the formulation 1B significantly reduces in 103 %* ($p<0.05$) the increase in MDA produced by the pollutant mixture. This means formulation 1B completely inhibits the oxidative stress produced by the contaminants.

**IL1-α**

IL1-α takes part in the immunologic response and inflammatory responses. The concentration of interleukins released in the culture medium is used to measure the inflammatory response induced by the exposure to pollutants, in order to assess the protective activity of formulation 1B.

As it can be seen in Figure 7, formulation 1B significantly reduces in 82 % * ($p<0.05$) the IL1-α amount released after the exposure to the pollutant mixture.

**AhR expression**

The aryl-hydrocarbon receptor (AhR) is a transcriptional factor involved in the molecular response subsequent to exposure to several compounds, including polycyclic aromatic hydrocarbons (PAHs), ozone and plant polyphenols (26, 22). In normal circumstances, AhR is in the cytoplasm bound to a protein complex consisting of HSP90, c-scr and other accompanying proteins. In the presence of the previously mentioned pollutants, AhR dissociates from the protein complex, is translocated to the nucleus where it dimerises with ARNT over promoting sequences which are specifically activated in response to xenobiotics. The target genes of AhR are cytochrome P450 (CYP) 1ª1, CYP1A2 and CYP1B1. In parallel, air pollutants, including the polycyclic aromatic hydrocarbons, are able to stimulate an increase in the ligand activity to the NF-kB DNA, which in turn is able to stimulate the gen that codifies AhR (27, 28).

AhR is expressed by several cell types, including keratinocytes, melanocytes, Langehans cells and T cells (29).

One of the mechanisms by which the air pollution causes skin damage is by AhR activation, as previously described.

As it can be seen in Figure 8, day 3 of pollutant exposure induces a significative increase of 60% ** ($p<0.01$) of the AhR expression. The application of formulation 1B causes a significant reduction of 87 % **($p<0.01$) in the AhR expression, as compared with the explants solely exposed to the pollutants.

**MT-1H expression**

The metallothionein protein (MT-1 H) is used as a marker for heavy metals exposure and oxidative stress.

Metallothioneins (MTs) are a group of proteins widely distributed in the tissues which regulate metal metabolism, free radical scavenging and also take part in immunologic reactions. Metallothionein 1 (MT-H1) is a unique protein stable under heat, with low molecular weight (6 kDa) and without aromatic aminoacids. Due to its great affinity to metal binding and to be induced by essential metals such as zinc and copper and other harmful metals such as cadmium and mercury, it is believed that it plays an important role in the essential metals homeostasis and in heavy metals detoxification.

As it can be seen in Figure 9, the MT-H1 marker did not increase in spite of the presence of pollutants, which suggests that formulation 1B exhibits a protective effect on the skin.

On exposure day 3, the pollutants caused an increase of 63 % **($p<0.01$) in the MT-H1 expression. The application of formulation 1B caused a significant reduction of 40 % **($p<0.01$) in the MT-H1 expression, as compared

with the explants solely exposed to the pollutants.

**EXAMPLE 4. Oral acute toxicity determination of formulation 1B (ZeroPollution).**

The objective of this study is to assess the oral acute toxicity of formulation 1B (ZeroPollution) by the "Acute Toxic Class Method" (limit test, OECD guideline no. 423) applied to Sprague-Dawley type female rats. The initial dose level was 2000 mg/kg body weight, given that it was considered likely it would not cause deaths. This method allows to classify formulation 1B (ZeroPollution) according to the Global Harmonised System (GHS) for the chemical products toxicity determination.

Summary of the study. The "limit test" is based in a procedure performed step by step with an initial dose of 2000 mg/kg body weight, carried out in 6 animals in two steps (three animals per step) The following doses would have been 300, 50 and 5 mg/kg body weight. After a 14 days observation period, the mortality due to formulation 1B administered in the first step is used to decide if the method continues with the following dose and to classify the substance according to an international classification. The following dose is not administered until the survival rate of the animals administered with the previous dose has been determined.

As no animals died during the observation period, it can be concluded that formulation 1B (ZeroPollution) can be classified in Category 5 or "Non classified" of the Global Harmonised Classification System for substances and mixtures.

**EXAMPLE 5. Instrumental clinical evaluation of the efficacy of a dietetic supplement that comprises the composition 1B of the invention (ZeroPollution) for its use in the prevention and/or treatment of the air pollution effects on the skin.**

The effect of the composition of the invention was studied in a monocentric, random, double-blind, placebo-controlled assay as protector from environmental pollutants after its use during 12 weeks in 100 women.

A cream for daily use was provided to all participants. The creams (placebo and composition 1B of the invention) were supplied with an anonymous packaging. Data were collected at assay weeks 2, 4, 8 and 12. The a) total antioxidant capacity in saliva and b) the oxidative skin damage were studied.

Furthermore, several parameters related to the skin aging were studied: a) skin hydration, b) skin luminosity, c) skin elasticity, d) transepidermal water loss, f) skin pigmentation, g) skin sebum content, and h) skin profilometry (wrinkles).

The participants were asked to respect the following requirements:

- To not use any cosmetic al least during two days before the start of the study;
- To not sunbath (naturally or with UV artificial sources) during all the study period;
- To only use the supplied product and to not use any other cosmetic.

The condition to withdraw from the study was the presence of adverse reactions or intolerance reactions in a elevated percentage of individuals. Possible withdrawals were not replaced.

The inclusion criteria were: healthy women, of 35 to 65 years old, 50 % Asiatic and 50 % Caucasic, 50 % with sensitive skin and outdoor work for at least two hours a day, that have not participated in a similar study over a suitable period, that accepted to not use products that might interfere with the product under study, that accepted to not sunbath (or to not use solar cabins) during the study unless they were suitably protected, that did not change of contraceptive method during the study, that were ready to comply with the study rules and that were ready to use the supplied product and neutral soap.

All individuals that did not meet the inclusion criteria were excluded, or those that had an atopic or hypersensitive skin, cosmetic allergies, allergies to soap or skin or sun protection medications, as well as individuals that had been subjected to a laser or chemical peeling treatment in the two year previous to the study, individuals that had used cosmetic or pharmaceutical products for the face on the day the study began, individuals with nutritional disorders, food allergies or intolerances, individuals with an altered immune system due to immunosuppressant diseases like AIDS, HIV or that used immunosuppressant medications, that were under pharmacological treatment that is know to interfere with the product or that has an effect on the metabolism, that suffer various serious diseases simultaneously, addiction to drugs, alcoholism, pregnant or breastfeeding women, individuals protected by law (under custody, hospitalised in a public or private institution, for a different reason to the research study, or jailed), that were not able to communicate or cooperate with the researchers due to language problems, intellectual development or cerebral function disorders, of for other reasons that the researchers identified.

Such medical occurrences with unfavourable symptoms or diseases temporarily associated with the product use were defined as adverse events, be it or not caused by said product use. The adverse events were monitored until

their resolution. The same dermatologic researcher was assigned to each individual during all the study. The product tolerance was studied in detailed manner, as well as each annoyance, and all possible intolerance reactions were informed, as well as any unexpected effect. In case that a secondary effect was classified as severe, the individual exclusion took place and said secondary effect was monitored until its resolution.

Each study individual was identified with a personal code that can not be associated with any other individual during the study.

According to the Helsinki declaration (1964), the individuals have the right to withdraw from the study at any time and for any reason, which will ne evaluated and classified. The researcher can also exclude any individual at any time for justified reasons. However, to avoid results interpretation problems in the case of an elevated number of withdrawals, the withdrawals will be avoided to the greatest possible extent. No individuals were replaced during the study.

All individuals with adverse events, be it in connection or not with the study, were monitored to determine their evolution, until the event was stabilised or resolved, even if the observation period was already finished.

The product was supplied in hard gelatine capsule form (Blue N°0) once a day 30 minutes before breakfast.

A suitable statistic model based on the data distribution was used, including comparison with the initial moment. Values of $p<0.05$ were considered statistically significant. The individuals will answer a questionnaire at weeks 4 and 12 of the study and a daily report (days 0 to 27).

The capsule with the active product comprised 250 mg of the composition of the invention (1B of example 1) and 120 mg of microcrystalline cellulose.

The placebo capsule comprised 250 mg of maltodextrin and 120 mg of microcrystalline cellulose.

The products were preserved at ambient temperature (less than 30 °C), protected form direct sunlight, heat and water.

A biostatistician generated, and kept in a safe place, a limited randomised list using the PASS 2008 software (PASS, LLC. Kaysville, UT, USA) in a Windows Server 2008 R2 Standard SP1 64bit Edition (Microsoft, USA). The randomisation product (or placebo) sequence was stratified using a 10% maximum deviation using 1:2 distribution ratios. The distribution was hidden in opaque and closed envelopes, indicating the treatment distribution based on the number assigned to each individual at the beginning of the study. An independent technician supplied the active or placebo according to the number assigned to each individual.

To evaluate the product efficacy, a subjective evaluation questionnaire was provided to each user at weeks 4 and 12 from the start of the study, and also the following measurements were performed:

<u>Skin hydration:</u>

Pollution alters the skin function as a barrier, causing that the skin turns dry and sensitive. Therefore, the skin hydration was evaluated, as the transepidermal water loss during the study.
The skin hydration assessment was measured in 5 points of the right cheek and the Corneometer® CM 825 method was used (Courage+Khazaka, electronic GmbH), based on the water dielectric constant. It uses a probe that measures capacitance changes in function of skin moisture content. An electric field penetrates in the outermost skin layers and determines the dielectricity.
Figure 10 shows that composition 1B of the invention provided a significant increase in skin hydration from the start of the study (measured as % change of hydration with respect to the start of the study, T0).
Moreover, Figure 11 shows that the composition 1B of the invention reinforced the epidermis barrier reducing in a significant way the transepidermal water loss (measured as % change of the hydration with respect to the start of the study, T0). At the end of the study, 82 % of the patients treated with the composition 1B of the invention perceived an improvement on the skin sensibility and 84 % of them perceived a protective feeling of the skin. Furthermore, 86 % of the patients treated with the composition 1B of the invention felt that their skin is more hydrated.

<u>Skin sebum assessment:</u>

Skin exposed to pollution secretes a greater sebum amount, causing, together with an increase in inflammation, a greater acne incidence.
The Sebumeter® apparatus (Sebumeter 815, Courage+Khazaka GmbH) as used. Said method uses photometry as principle. Skin sebum was collected in a synthetic tape of 64 $mm^2$, contained in a cassette, later on, the cassette measurement head was inserted into the apparatus opening, in which a photoelectric cell measure the transparency. The light transmission represents the sebum content in the measured surface area. A microprocessor calculated the result, indicated as $\mu g$ sebum/$cm^2$. Figure 12 shows how the composition 1 B of the invention reduced the sebum secretion produced under pollution exposure. Moreover, 82 % of the treated individuals perceived an improvement in skin imperfections, such as pore dilatation or in the black heads decrease.

Skin firmness and elasticity:

The skin firmness and elasticity measurements were carried out on the cheek skin using a suction method that creates a negative pressure that mechanically deforms the skin (Cutometer® method). A 450 mbar negative pressure was created attracting the skin on the probe opening during 2 seconds and releasing it again after 2 seconds. The penetration depth in the probe was determined, by an optical detection system (without contact). The optical system consists of a light source and a light receptor, as well as two opposed prisms that project light from the transmitter to receptor. The light intensity changes depending on the skin penetration depth. The skin resistance to negative pressure and its ability to return to its initial position is represented in curves (penetration depth in mm/time unit) in real time during the assessment. The apparatus used is a MPA 580 Cutometer® (Courage+Khazaka, electronic GmbH). The selected biomechanical indicators were:

R2: total skin elasticity. Portion between maximum amplitude and skin capacity to redeformation (brut elasticity)

R0: maximum skin extensibility (tone/firmness) The lower the parameter the higher the skin resistance to deformation.

The lower the parameter R0 the higher the skin resistance to deformation. In this way, a decrease in the R0 value implies, in absolute values, an increase in skin firmness.

Figure 13 shows how the composition 1B of the invention produces a significative increase in total skin elasticity in comparison with the placebo use (data taken at 14, 28, 56 and 84 days of use).
The composition 1B of the invention also produced a significant increase in skin firmness (measured as RO absolute value reduction, as representative of the skin firmness increase), as it can be appreciated in Figure 14, in data taken at 14, 28, 56 and 84 days of use.

Furthermore, it was observed in the subjective evaluation questionnaire at the end of the study that 82 % of the individuals that took the composition 1B of the invention perceived an increase in skin firmness and a healthier complexion.

Skin colour and luminosity:

Pollution is also responsible of an increase in pigmentation disorders. In fact, traffic originated pollution, or soot, might increase skin spots up to 20 %.

Skin luminosity and skin colour were measured using a CM 700D spectrophotometer/colorimeter (Konica Minolta). The ITA value (from its English acronym, Individual Topology Angle) defines the skin pigmentation degree and it was calculated using the L and b values (form the L, a, b colour space) obtained with the spectrophotometer, with the formula ITA=[ArcTan(L-50)/b]180/$\pi$

Skin luminosity was defined using the brightness value that was defined as the proportion of reflected to dispersed light (the greater the reflected light the greater the brightness, whereas more wrinkled surfaces disperse more light, resulting in smaller brightness values).

The ITA value was measured in a hyperpigmented area, whereas the luminosity was measured on the cheek.

The skin pigmentation content was measured with the MX18 Mexameter (Courag+Khazaka). The Mexameter® MX 18 probe emits at three specific wavelengths. A receptor measures the light reflected by the skin. Given that the emitted light amount is known, the light absorbed by the skin could be calculated. The melanin is measured at specific wavelengths.

Figure 15 reflects the increase (in %) with respect to the start of the study in skin luminosity (measured as brightness value on the cheek) after 14, 28, 56 and 84 days of use of the composition 1B of the invention versus the placebo use.

Figure 16 represents the increase (in %) of the ITA value measured in the hyperpigmentation area after the use of the composition 1 B of the invention versus the placebo use.

As it can be appreciated in Figures 15 and 16, the use of the composition 1B of the invention produced a more luminous skin, whereas the spot colouring significantly decreased.

Skin perfilometry (roughness):

Epidemiologic studies evidence that the exposure to environmental pollution is associated with a greater wrinkle manifestation degree.

The smoothness (ra parameter) and the wrinkles depth in the crow's foot area were measures, using a microtopographic 3D imaging system (Primoslite GFMesstechnik, GmbH). The skin surface was reconstructed using an algorithm to generate 3D images. The individuals repositioning was ensured using a repositioning apparatus (Canfield

Scientific); whereas before/after image coupling was ensured by a overlapping characteristic of the image analysis software. As it is appreciated in Figures 17 and 18, the use of composition 1B of the invention produced a softer and more uniform skin, whereas the wrinkles and lines depth significantly decreased.

Biochemical analysis

The following characteristics were measured using standard biochemical methods: Total antioxidant capacity by FRAP (from its English acronym Ferric Reducing Antioxidant Power) and the MDA assay (in skin exfoliation).

Skin exfoliation

Skin layers were taken using Corneofix® sheets (Caurage+Khazaka electronic GmbH). This technique allows to collect several layers of the *stratum corneum.* Layers 2 and 3 were collected and next were preserved at -80°C for its subsequent FRAP analysis.

Malonaldehyde (MDA) and 4-hydroxynonenal are the two major products of lipid peroxidation, that is why their concentration in a biological system can be used as a lipo-peroxide damage index. In order to determine the lipo-peroxide levels the Erdelmeier *et al.* (1998) method based on the reacting capacity of N-methyl-2-phenylindol chromogen with MDA at 45 °C and acid pH to produce a chromophore with a absorption peak at 586 nm:

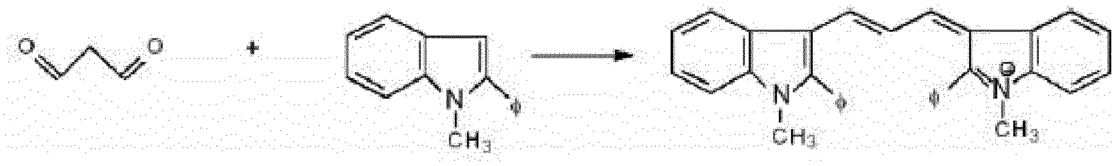

The lipo-peroxide levels were measured after the unstable hydroperoxides decomposition induction, produced by oxidative processes through the use of a pro-oxidative agent ($CoSO_4$ 500 $\mu$M).

The composition 1B of the invention (ZeroPollution) reduced the MDA production, as a measure of the skin peroxidation at 28, 56 and 84 days of its use, as it is shown in Figure 19.

FRAS (Saliva ferric antioxidant reductor):

The FRAS assay is a direct measure of the reducing power of a system and is an indirect value of the organism capacity to resist oxidative damage. The reduction at acid pH of the TPTZ-Fe(II) complex to the Fe(III) one is characterised by an intense blue colour. The reaction is monitored measuring the solution absorbance at 595 nm. The collected absorbances are compared to the ones of a standard curve of known Fe(II). The results are directly proportional to the total reducing capacity of the mixture antioxidants.

Figure 20 shows how the composition 1B of the invention (ZeroPollution) produced a FRAS increase, thus showing antioxidant capacity at 28 days and even greater at 84 days of its use.

The results of these measurements is collated, next, in Table 3:

Table 3

| | Placebo | | | | Composition 1B | | | |
|---|---|---|---|---|---|---|---|---|
| | T14 | T28 | T56 | T84 | T14 | T28 | T56 | T84 |
| Skin hydration | +1,2%$^{ns}$ | +3.5%$^{s}$ | +5,8%$^{s}$ | +5,3%$^{s}$ | 5,7%$^{s}$ | 9,0%$^{s}$ | 12,3%$^{s}$ | 14,8%$^{s}$ |
| Transdermal wáter loss | -1,5%$^{s}$ | -2,8%$^{s}$ | -2,7%$^{s}$ | -2,3%$^{s}$ | -3,8%$^{s}$ | -6,2%$^{s}$ | -9,2%$^{s}$ | -10,1%$^{s}$ |
| Wrinkles Depth | 0,3%$^{ns}$ | -3,1%$^{ns}$ | -4,2%$^{s}$ | -2,2%$^{s}$ | -10,6%$^{s}$ | -14,5%$^{s}$ | -18,0%$^{s}$ | -20,3%$^{s}$ |
| Ra* parameter | +1,1%$^{ns}$ | -0,5%$^{ns}$ | -1,5%$^{ns}$ | -0,4%$^{ns}$ | -3,4%$^{s}$ | -4,4%$^{s}$ | -6,2%$^{s}$ | -7,6%$^{s}$ |
| Skin elasticity: R2 | +0,9%$^{s}$ | +0,3%$^{ns}$ | +0,1%$^{ns}$ | +0,9%$^{ns}$ | +2,9%$^{s}$ | +6,1%$^{s}$ | +7,1%$^{s}$ | +8,0%$^{s}$ |
| Skin firmness: R0** | -0,9%$^{ns}$ | -0,1%$^{ns}$ | +0,3%$^{ns}$ | -1,0%$^{ns}$ | -3,8%$^{s}$ | -4,7%$^{s}$ | -6,7%$^{s}$ | -7,1%$^{s}$ |
| Skin colour (ITA angle) | +3,4%$^{ns}$ | +4,2%$^{s}$ | +5,4%$^{s}$ | +6,6%$^{s}$ | +8,2%$^{s}$ | +12,1%$^{s}$ | +13,9%$^{s}$ | +16,4%$^{s}$ |

(continued)

| | Placebo | | | | Composition 1B | | | |
|---|---|---|---|---|---|---|---|---|
| | T14 | T28 | T56 | T84 | T14 | T28 | T56 | T84 |
| Skin luminosity (brightness) | +2,2%$^{s}$ | +3,3%$^{s}$ | +3,6%$^{s}$ | +4,2%$^{s}$ | +8,4%$^{s}$ | +14,6%$^{s}$ | +18,6%$^{s}$ | +20,0%$^{s}$ |
| Skin sebum content | -1,3%$^{ns}$ | -3,3%$^{s}$ | -5,9%$^{s}$ | -6,4%$^{s}$ | -3,9%$^{s}$ | -5.1%$^{s}$ | -9,5%$^{s}$ | -10,9%$^{s}$ |
| MDA*** assay | -- | -2,6%$^{ns}$ | -3,1 %$^{ns}$ | -2,6%$^{ns}$ | -- | -5,2%$^{s}$ | -7,5%$^{s}$ | -10,0%$^{s}$ |
| FRAS**** | -- | +3,8%$^{ns}$ | -- | +5,7%$^{ns}$ | -- | +21,7%$^{s}$ | -- | +35,0%$^{s}$ |

*The Ra parameter is related with skin smoothness. A decrease in RA values can be expressed in absolute value as skin smoothness increase.
** The R0 parameter is related with the skin firmness. A decrease of the R0 parameter can be expressed in absolute values as a skin firmness increase.
***The MDA production is a measure of the oxidative damage in the epidermis.
****The FRAS value is related with the systemic antioxidant capacity measured in saliva samples.
s: statistically significant vs. T0 / ns: not statistically significant vs. T0

## REFERENCES

(1) Krutmann J. et al. Environmentally induced (extrinsic) skin aging. Hautarzt.2016 Feb; 67(2):99-102.

(2) Morgenstern, V. et al. Atopic diseases, allergic sensitization, and exposure to traffic-related air pollution in children. Am. J. Respir. Crit. Care Med. 177, 1331-1337 (2008).

(3) Song, S. et al. Acute health effects of urban fine and ultrafine particles on children with atopic dermatitis. Environ. Res. 111, 394-399 (2011).

(4) Kim, J. et al. Symptoms of atopic dermatitis are influenced by outdoor air pollution. J. Allergy Clin. Immunol. 132, 495-499 (2013).

(5) Van Den Bogaard, E.H. et al. Coal tar induces AHR-dependent skin barrier repair in atopic dermatitis. J. Clin. Invest. 123, 917-927 (2013).

(6) Kim, H.O., Kim, J.H., Chung, B.Y., Choi, M.G. ; Park, C.W. Increased expression of the aryl hydrocarbon receptor in patients with chronic inflammatory skin diseases. Exp. Dermatol. 23, 278-281 (2014).

(7) DiMeglio, P. et al. Activation of the aryl hydrocarbon receptor dampens the severity of inflammatory skin conditions. Immunity 40, 989-1001 (2014).

(8) Jean Krutmann, Dominique Moyal, Wei Liu, Sanjiv Kandahari, Geun-Soo Lee, Noppakun Nopadon, Leihong Flora Xiang y Sophie Seité. "Pollution and acne: is there a link?" Clinical, Cosmetic and Investigational Dermatology. May 2017

(9) Ju Q, Zouboulis CC, Xia L. Environmental pollution and acne: Chloracne. Dermato-endocrinology. 2009;1(3):125-128

(10) Vierkoler A et al. Airborne particle exposure and extrinsic skin aging, J. Invest. Dermatol. 130 (12) (2010) 2719-2726.

(11) Hüls A et al. Traffic-Related Air Pollution Contributes to Development of Facial Lentigines: Further Epidemiological Evidence from Caucasians and Asians. J. Invest. Dermatol. 2016 May; 136(5):1053-6.

(12) Weng, Zuyi et al. Quercetin Is More Effective than Cromolyn in Blocking Human Mast Cell Cytokine Release

and Inhibits Contact Dermatitis and Photosensitivity in Humans. Ed. Christian Taube. PLoS ONE7.3 (2012): e33805. PMC. Web. 15 Sept. 2017.

(13) Sim, H.S.S. and S.S., Acetoside inhibits a-MSH- induced melanin production in B16 melanoma cells by inactivation of adenyl cyclase. Journal of Pharmacy and Pharmacology 2009. 61: p. 1347-1351.

(14) Young-Ok Sona, S.-A.L.e.a., Acteoside inhibits melanogenesis in B16F10 cells through ERK activation and tyrosinase down-regulation. Journal of Pharmacy and Pharmacology 2011. 63: p. 1309-1319.

(15) Mancebo SE, Wang SQ. Recognizing the impact of ambient air pollution on skin health. J Eur Acad Dermatol Venereol. 2015 Dec;29(12):2326-32.

(16) Mulero-Navarro S, Fernandez-Salguero PM. New Trends in Aryl Hydrocarbon Receptor Biology. Frontiers in Cell and Developmental Biology.

(17) Abel J, Haarmann-Stemmann T. An introduction to the molecular basics of aryl hydrocarbon receptor biology. Biol Chem. 2010 Nov;391(11):1235-48.

(18) Krutmann J, Jux B, Luecke S, Fritsche E, Abel J, Essel C, Rannug A. Involvement of arylhydrocarbon receptor (AhR-) signaling in skin melanogenesis. J Invest Dermatol 2008;128:S22

(19) Morita A, Torii K, Maeda A, Yamaguchi Y. Molecular basis of tobacco smoke-induced premature skin aging. J Investig Dermatol Symp Proc. 2009 Aug;14(1):53-5.

(20) Murphy KA, Villano CM, Dorn R, White LA. Interaction between the aryl hydrocarbon receptor and retinoic acid pathways increases matrix metalloproteinase-1 expression in keratinocytes. J Biol Chem. 2004 Jun 11;279(24):25284-93.

(21) Luecke S, Backlund M, Jux B, Esser C, Krutmann J, Rannug A. The aryl hydrocarbon receptor (AHR), a novel regulator of human melanogenesis. Pigment. Cell Melanoma Res. 2010 Dec;23(6):828-33.

(22) Afaq F, Zaid MA, Pelle E, Khan N, Syed DN, Matsui MS, Maes D, Mukhtar H. Aryl hydrocarbon receptor is an ozone sensor in human skin. J Invest Dermatol. 2009 Oct;129(10):2396-403.

(23) Ono Y, Torii K, Fritsche E, Shintani Y, Nishida E, Nakamura M, Shirakata Y, Haarmann-Stemmann T, Abel J, Krutmann J, Morita A. Role of the aryl hydrocarbon receptor in tobacco smoke extract-induced matrix metalloproteinase-1 expression. Exp. Dermatol. 2013 May 22(5):349-53.

(24) Hidaka T, Ogawa E, Kobayashi EH, Suzuki T, Funayama R, Nagashima T, Fujimura, T, Aiba S, Nakayama K, Okuyama R, Yamamoto M. The aryl hydrocarbon receptor AhR links atopic dermatitis and air pollution via induction of the neurotrophic factor artemin. Nat Immunol. 2017 Jan;18(1):64-73

(25) Janero D.R. Malondialdehyde and thiobarbituric acid-reactivity as diagnostic indices of lipid peroxidation and peroxidative tissue injury. Free Radic Biol Med. 1990; 9(6):515-40.

(26) Haarmann-Stemmann T, Abel J, Fritsche E, Krutmann J. The AhR-Nrf2 pathway in keratinocytes: on the road to chemoprevention. J Invest Dermatol. 2012 Jan;132(1):7-9.

(27) Champion S. et al., Activation of the NFκB Pathway Enhances AhR Expression in Intestinal Caco-2 Cells, ISRN Toxicology Volume 2013 (2013).

(28) Tauchi et al., 2005. Constitutive Expression of Aryl Hydrocarbon Receptor in Keratinocytes Causes Inflammatory Skin Lesions, Molecular and cellular biology, Nov. 2005, p. 9360-9368.

(29) Furue et al., 2014. Role of AhR/ARNT system in skin homeostasis Arch Dermatol Res. 306:769-779

## Claims

1. Composition for the treatment of the air pollution effects on the skin **characterised in that** said composition comprises diterpenes, oleuropein, verbascoside, quercetin and hydroxytyrosol.

2. Composition according to claim 1 comprising between 25 % and 40 % weight of a rosemary extract providing a minimum diterpenes content of 4.5 % weight of the final formula, between 20 % to 40 % weight of a olive leaves or fruits extract providing a minimum oleuropein content of 4.5 % weight and a minimum hydroxytyrosol content of 1.5 % of the final formula, between 20 % and 35 % weight of a *Lippia Citriodora* extract providing a minimum verbascoside content of 6.5 % weight of the final formula and between 5 % and 25 % weight of a *Sophora Japonica* extract providing a minimum quercetin content of 3.5 % of the final formula.

3. Composition according to claim 1 or claim 2 comprising an individual weight ratio of approximately 2.5 to 5 of diterpenes, oleuropein, verbascoside and quercetin to hydroxytyrosol, respectively.

4. Composition according to claim 3 wherein the diterpenes to hydroxytyrosol weight ratio is around 3, the oleuropein to hydroxytyrosol weight ratio is around 3, the verbascoside to hydroxytyrosol weight ratio is around 4.5 and the quercetin to hydroxytyrosol weight ratio is around 2.5.

5. Composition according to any preceding claim comprising a *Sophora Japonica* extract with a minimum quercetin content of 40 % weight.

6. Composition according to any preceding claim comprising carnosic acid.

7. Composition according to any preceding claim comprising a rosemary extract with a minimum diterpenes content of 15% weight.

8. Composition according to any preceding claim comprising an olive extract with a minimum oleuropein content of 16 % weight.

9. Composition according to any preceding claim comprising an olive extract with a minimum hydroxytyrosol content of 5 % weight.

10. Composition according to any preceding claim comprising a *Lippia citriodora* extract with a minimum verbascoside content of 25 % weight.

11. Composition according to any of claims 1 to 10 for its use in the prevention of premature skin aging associated to air pollution effects.

12. Dose of between 0.1 to 1000 milligrams of the composition according to claim 10.

13. Dose of 250 milligrams of the composition according to claim 10.

14. Formulation for its parenteral, transdermal, oral or topical administration that comprises a composition according to any of claims 1 to 10.

15. Dietetic supplement comprising a composition according to any of claims 1 to 10.

16. Nutricosmetic comprising a composition according to any of claims 1 to 10.

17. Composition according to any of claims 1 to 10 for its use as a medicament.

18. Composition according to any of claims 1 to 10 for its use in the prevention and/or treatment of the skin affections caused by environmental pollution.

19. Composition for use, according to claim 18, wherein the skin affections caused by environmental pollution are selected from inflammation, acne, chronic dermatitis and psoriasis.

**20.** Composition for use, according to any of claims 17 to 18, wherein said composition is orally administered in a dose between 0.1 to 1000 mg a day.

**21.** Use of the composition according to any of claims 1 to 10 as cosmetic.

**22.** Use according to claim 21 for cosmetically alleviating the environmental pollution aesthetic effects on the skin.

**23.** Use according to claims 21 or 22 as cosmetic with antioxidant, anti-inflammatory effects, taking this effect not as a therapeutic treatment but as merely aesthetic, antiaging and whitening on the skin.

**24.** Use according to any of claims 21 to 23 wherein said composition is orally administered in a dose between 0.1 to 1000 mg a day.

Figure 1

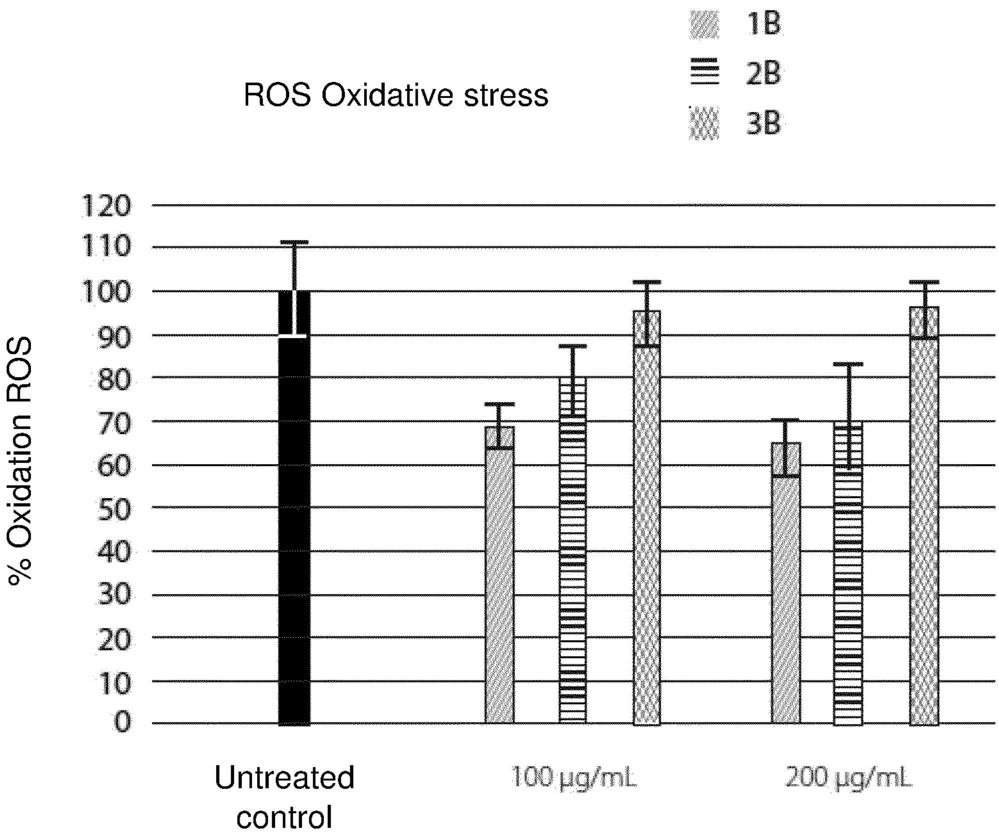
ROS Oxidative stress
1B
2B
3B
% Oxidation ROS
120
110
100
90
80
70
60
50
40
30
20
10
0
Untreated control
100 µg/mL
200 µg/mL

Figure 2A

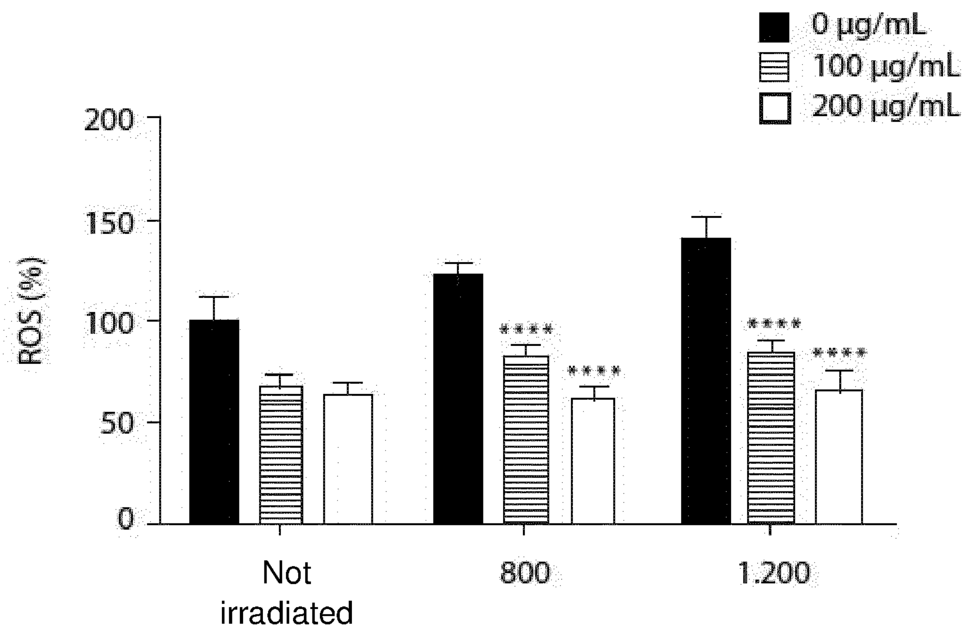


Figura 2B

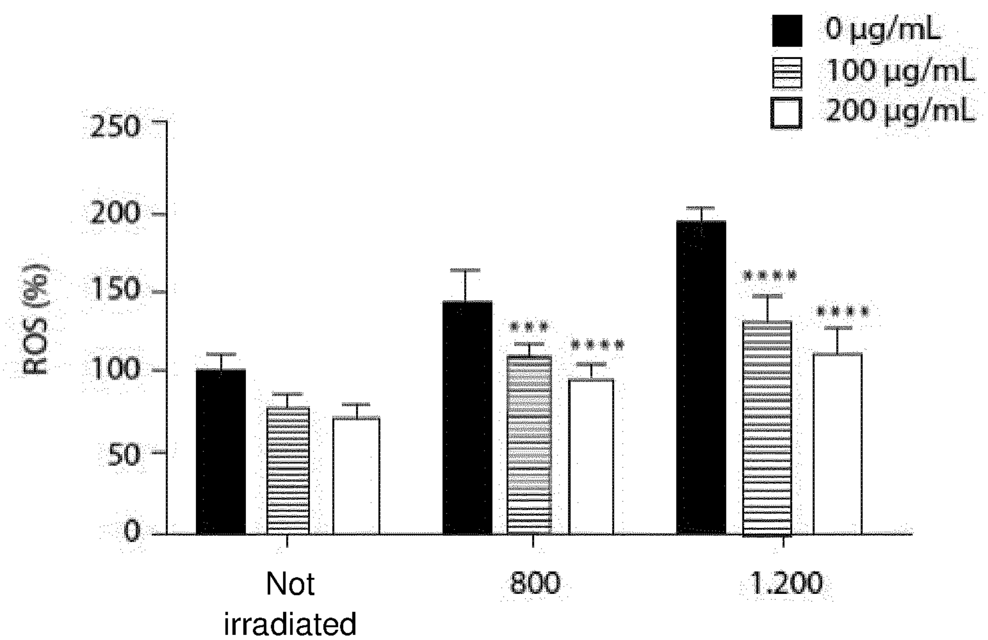

Figure 2C

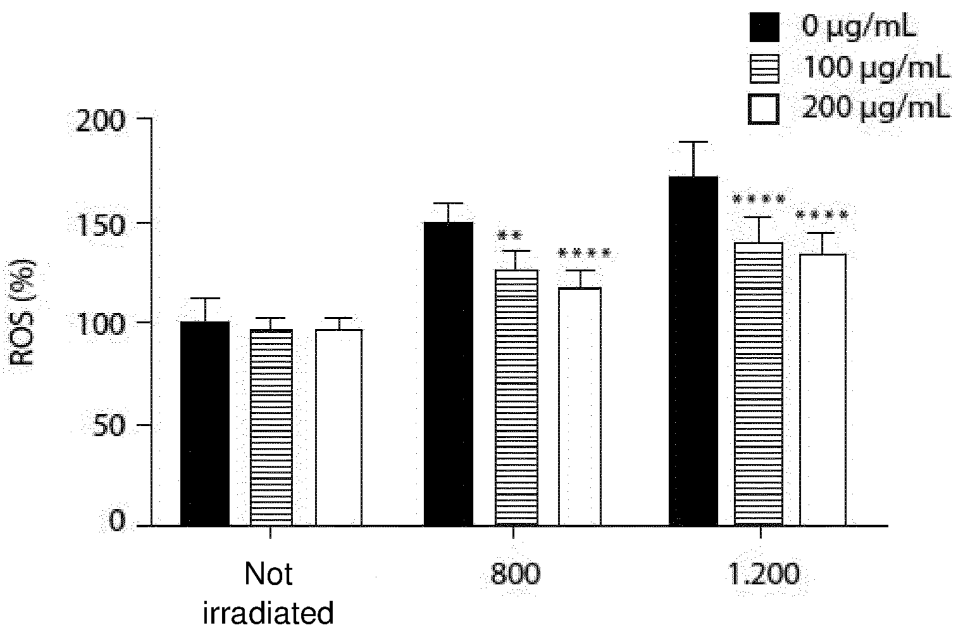


Figure 3

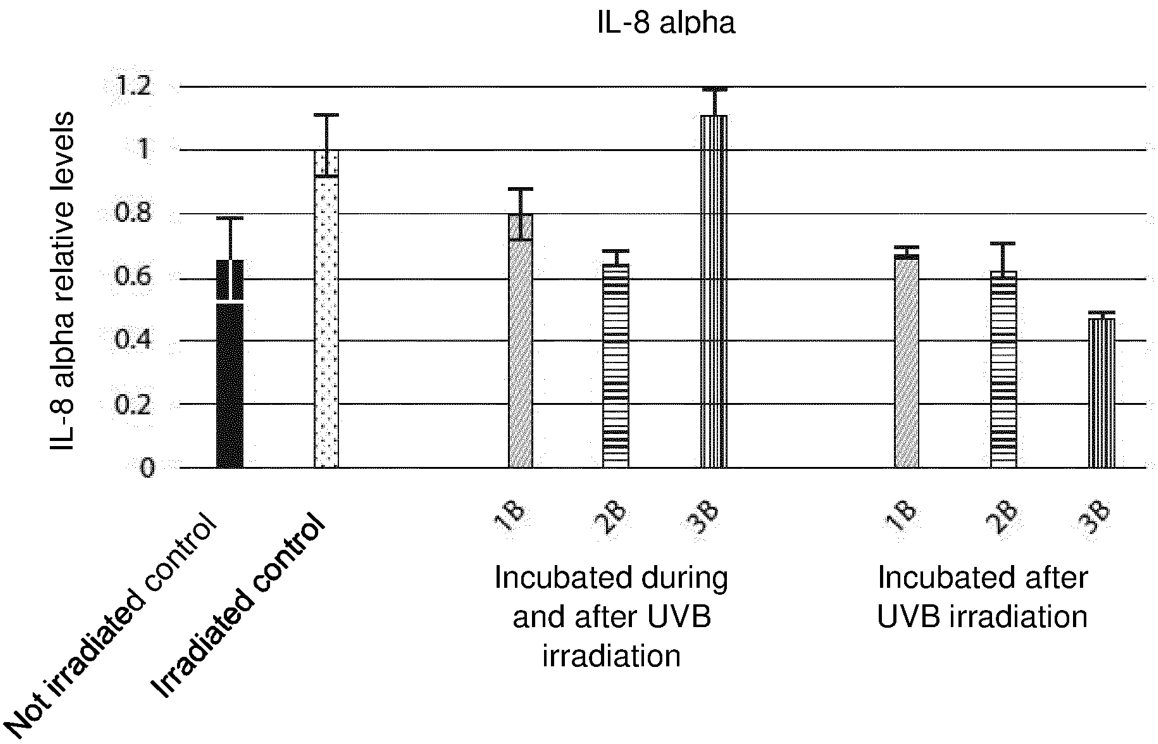

Figure 4A

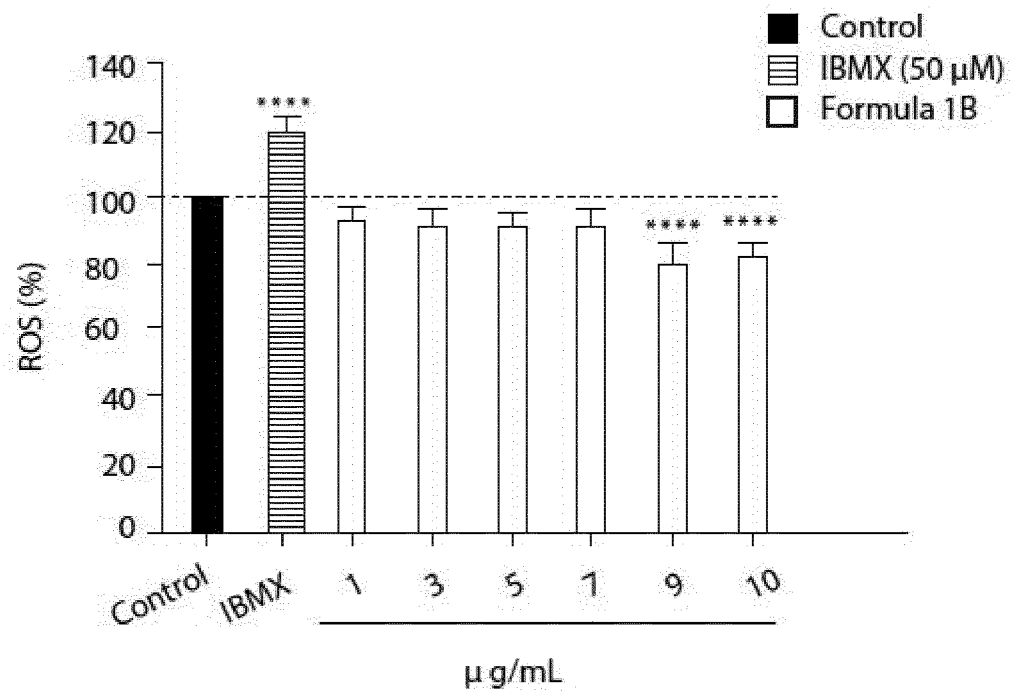


Figure 4B

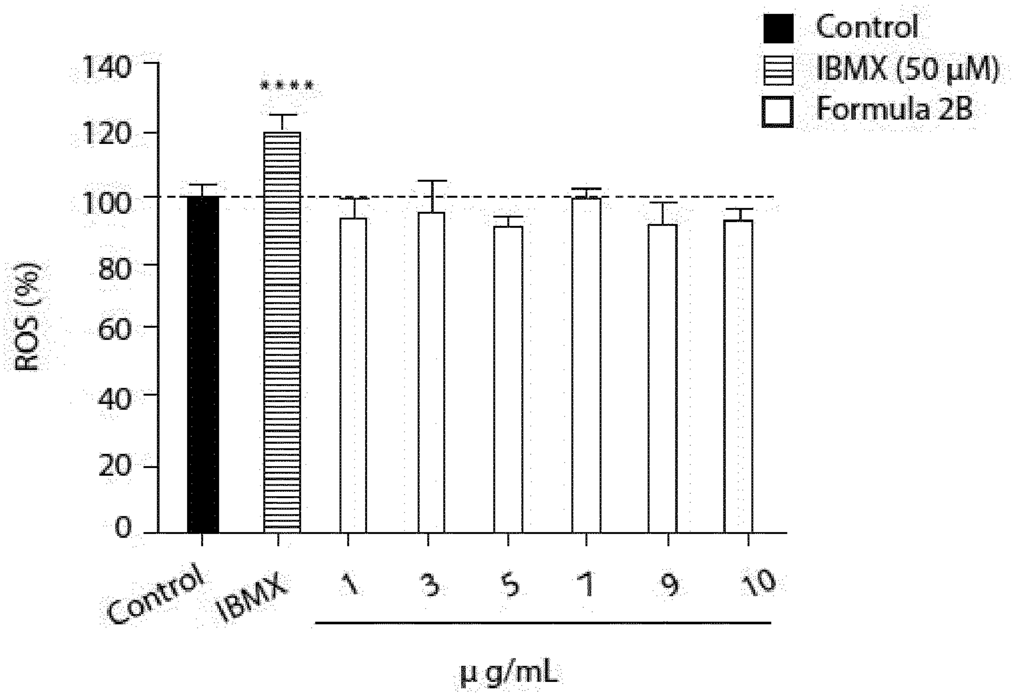

Figure 4C

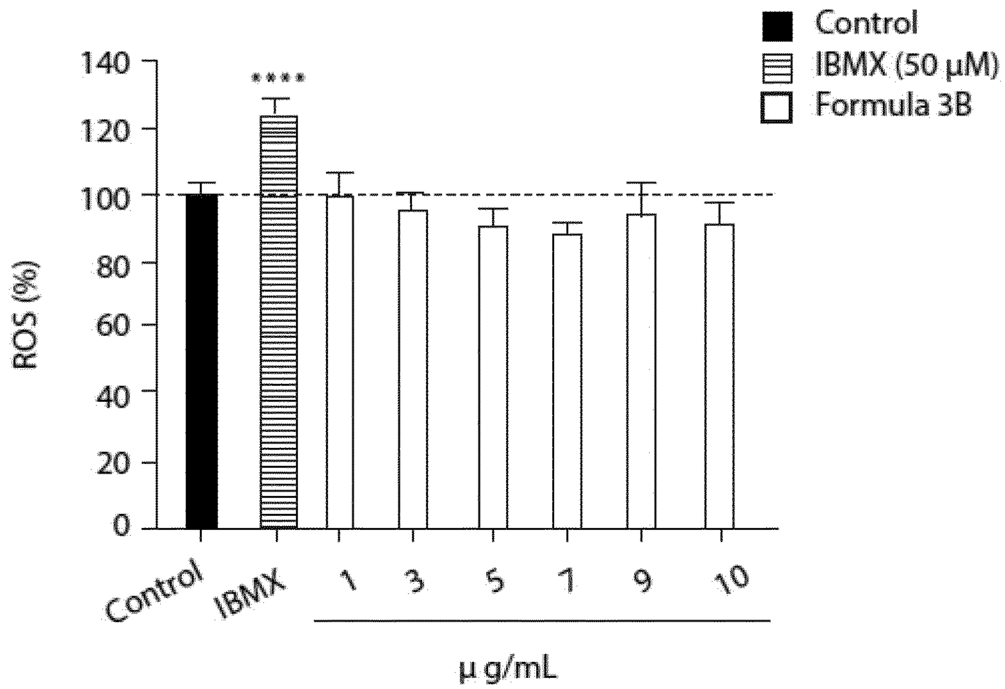


Figure 5

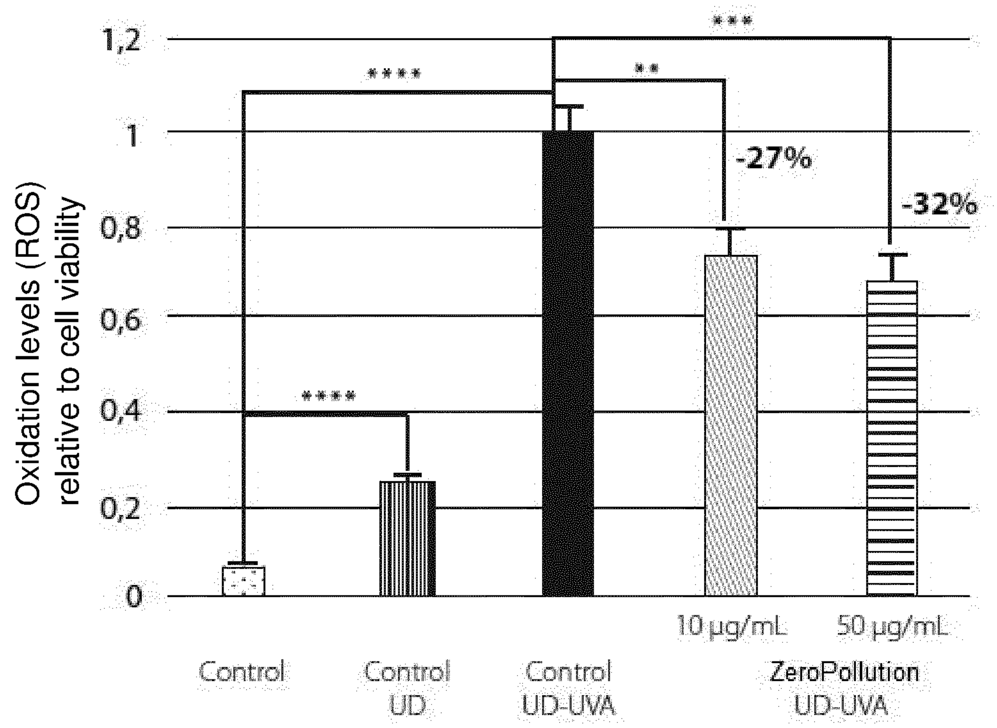

Figure 6

Malonaldehyde (MDA) concentration increase in the skin explants after exposure to pollutant agents

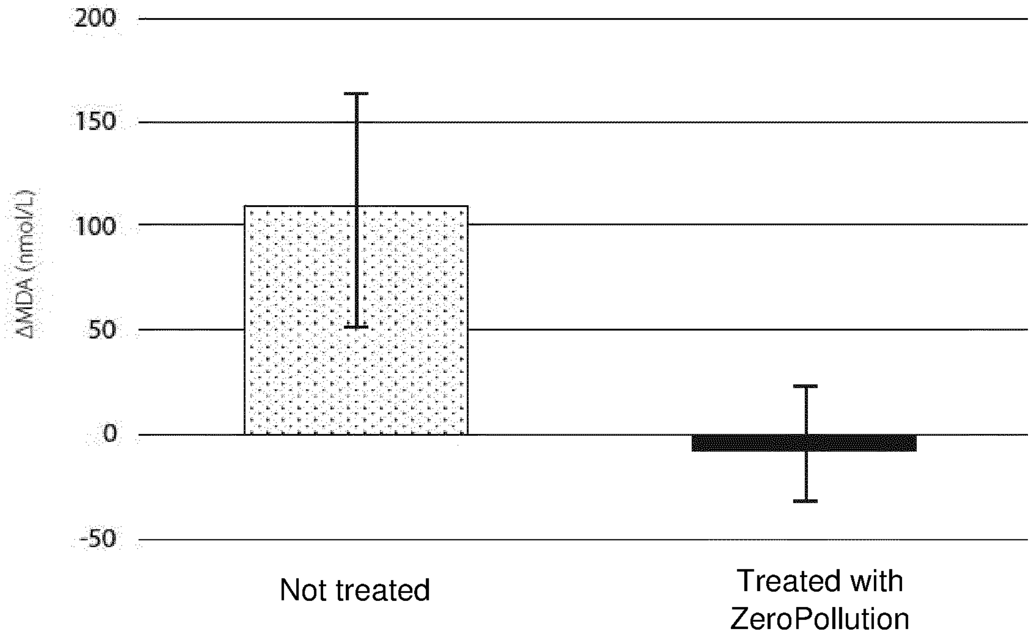


Figure 7

IL-1 alpha concentration released to the culture medium

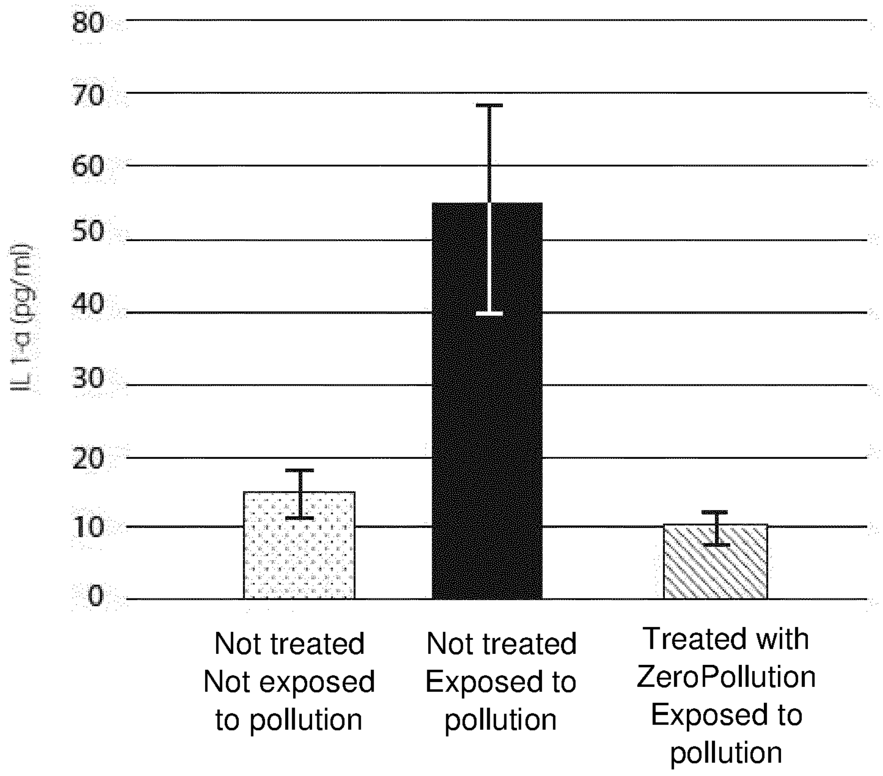

Figure 8

% explant epidermis positive area to the aryl hydrocarbon receptor (AhR)

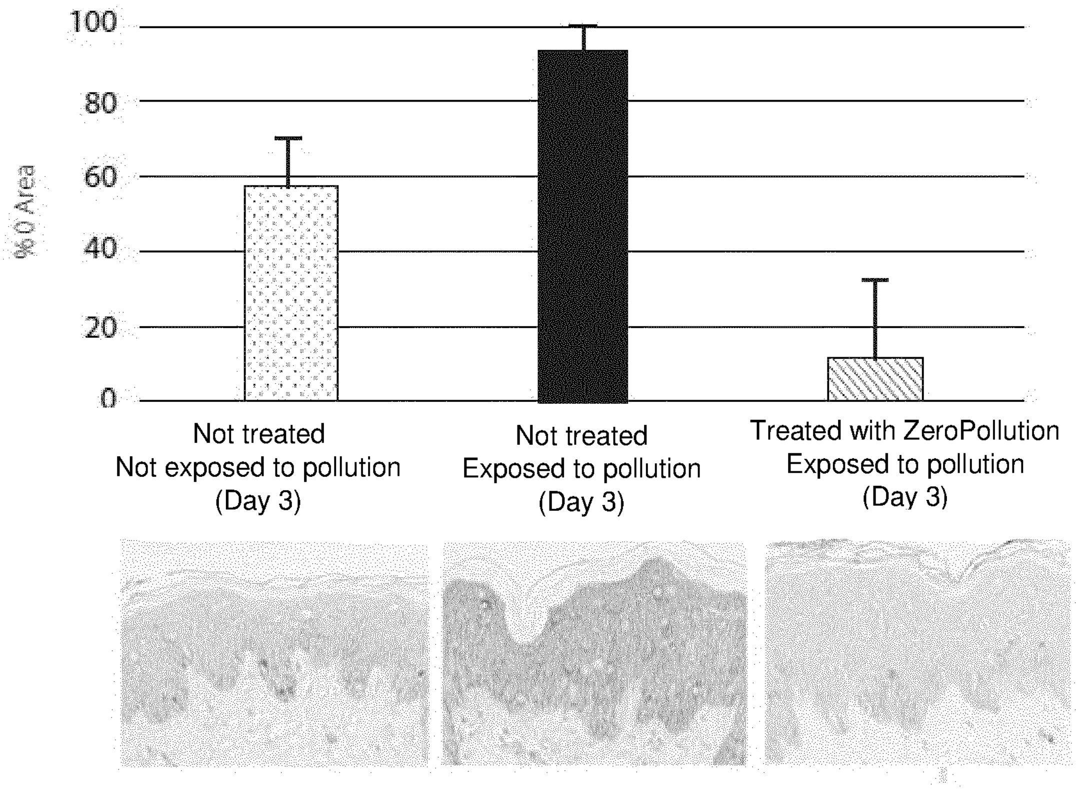

Figure 9

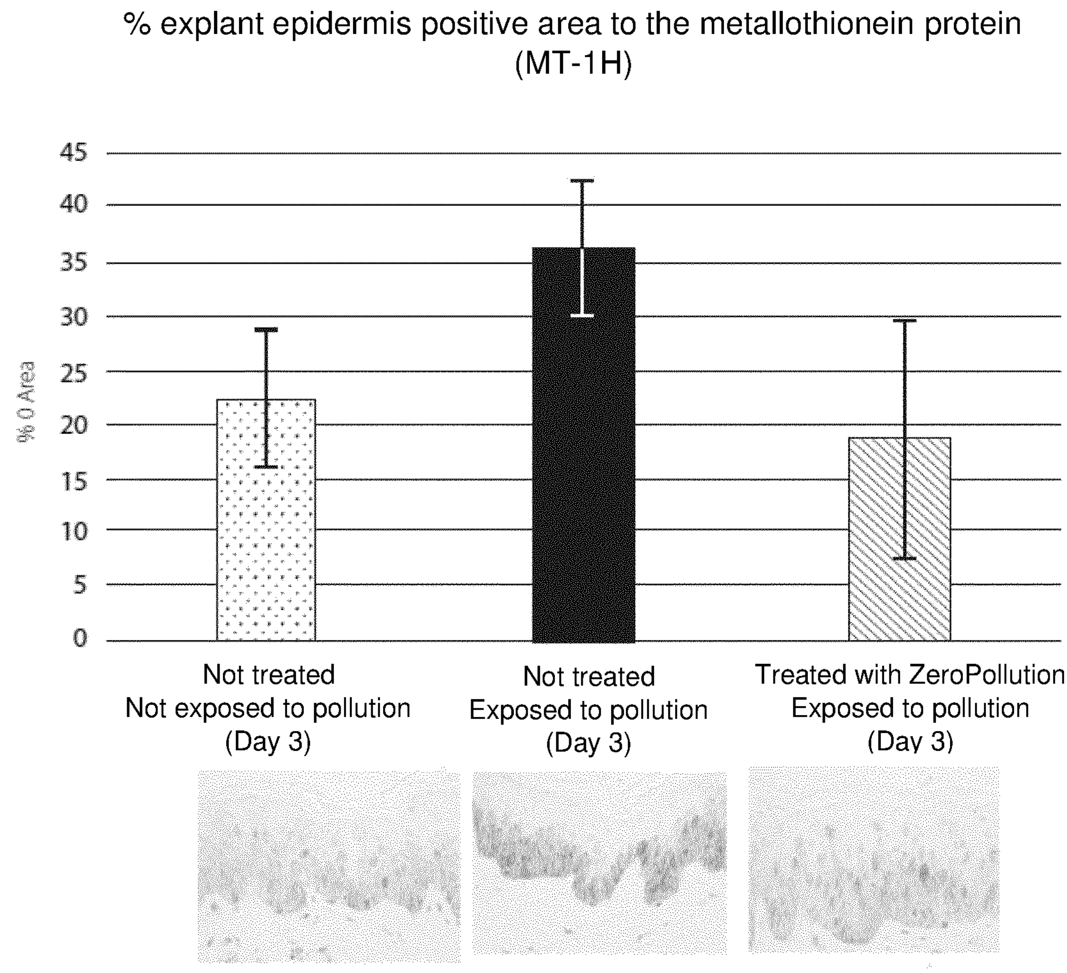
% explant epidermis positive area to the metallothionein protein (MT-1H)
45
40
35
30
25
20
15
10
5
0
% 0 Area
Not treated
Not exposed to pollution
(Day 3)
Not treated
Exposed to pollution
(Day 3)
Treated with ZeroPollution
Exposed to pollution
(Day 3)

Figure 10

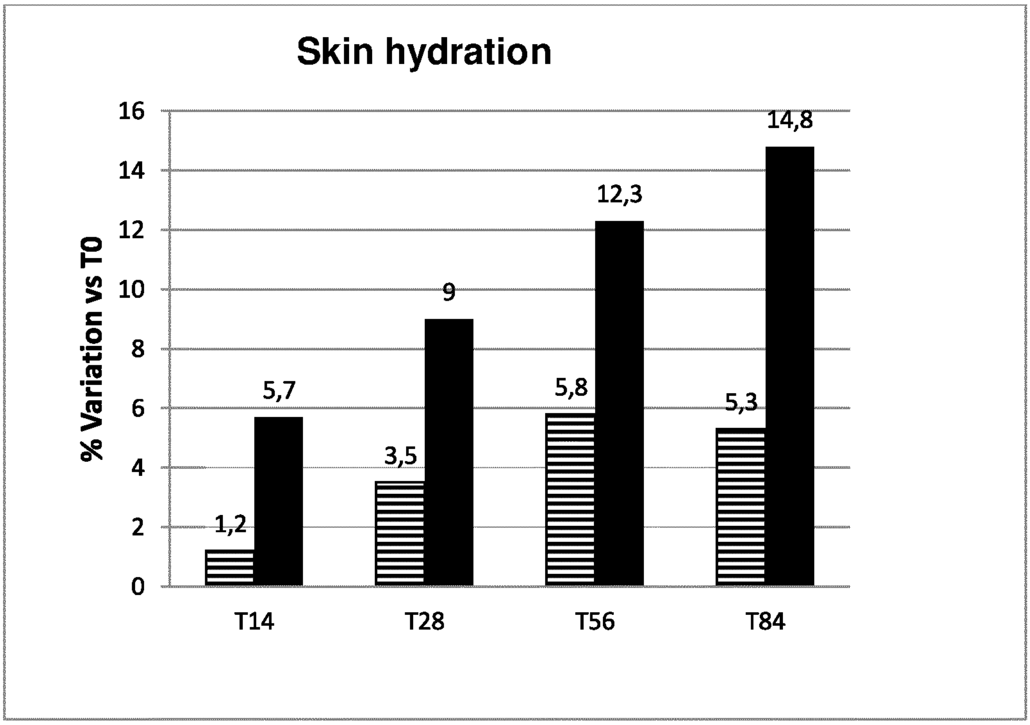


Figure 11

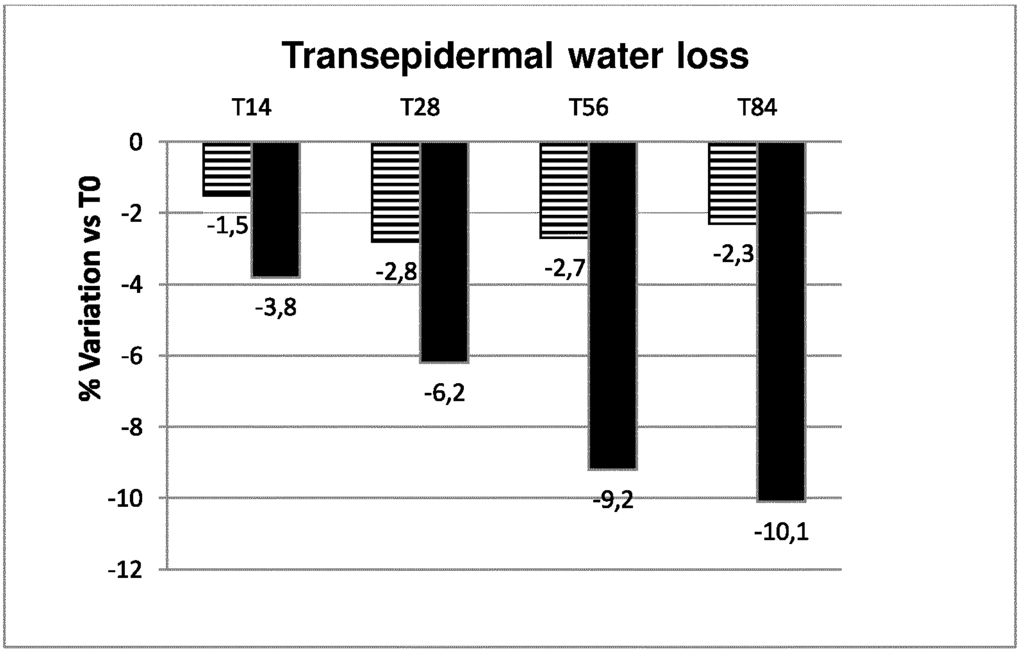

Figure 12

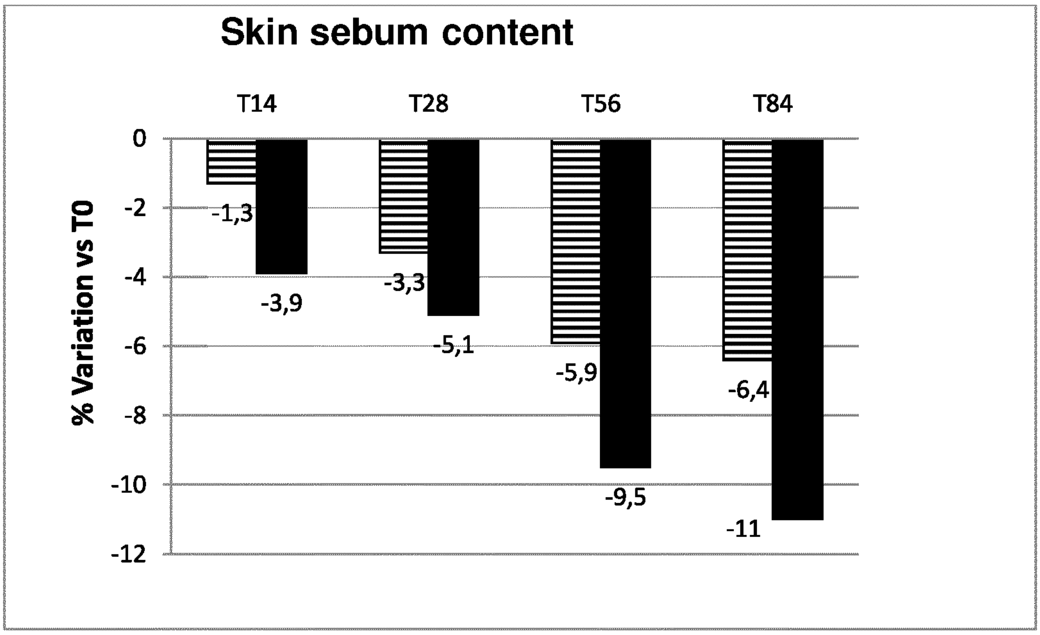


Figure 13

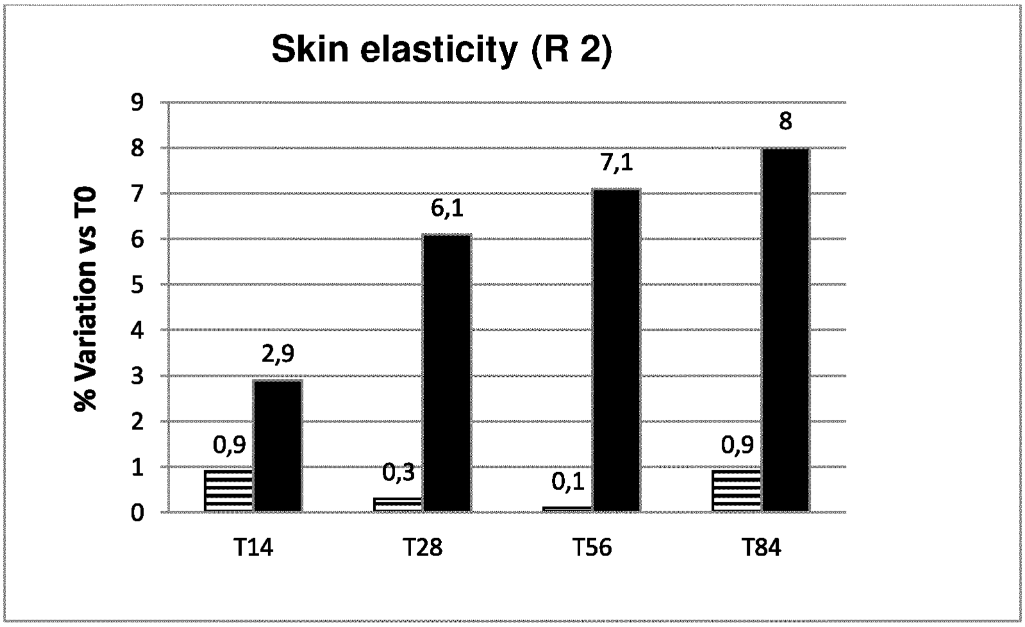

Figure 14

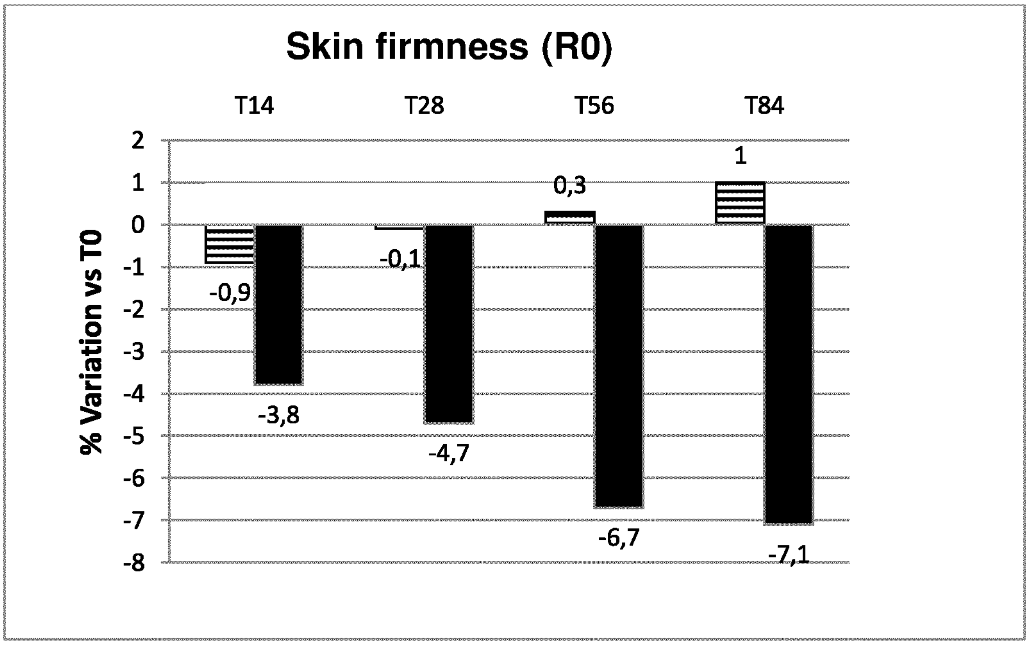


Figure 15

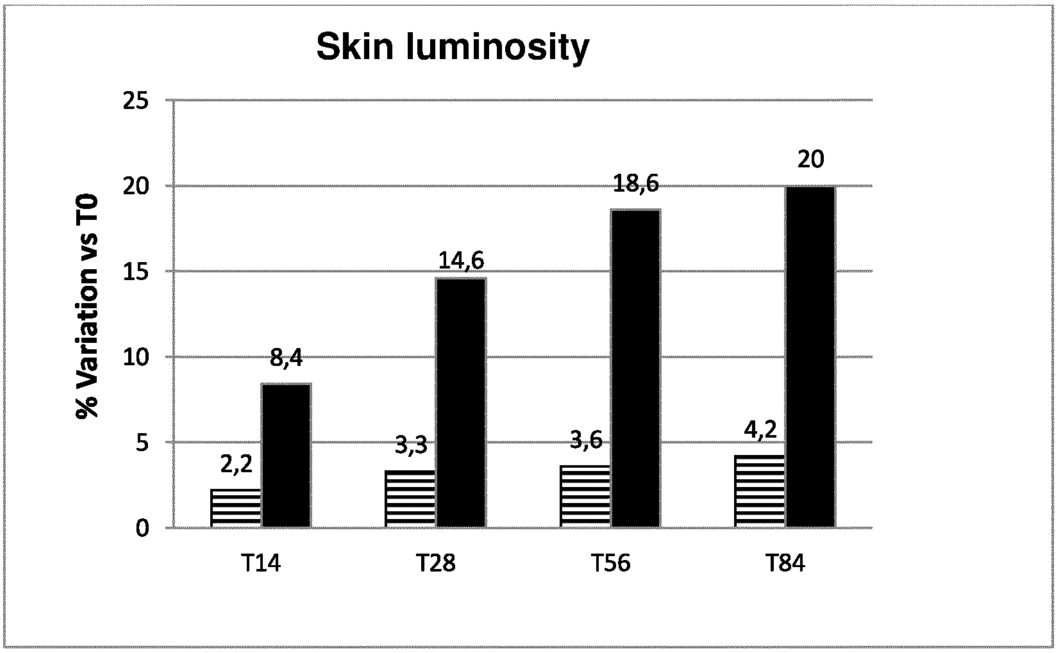

Figure 16

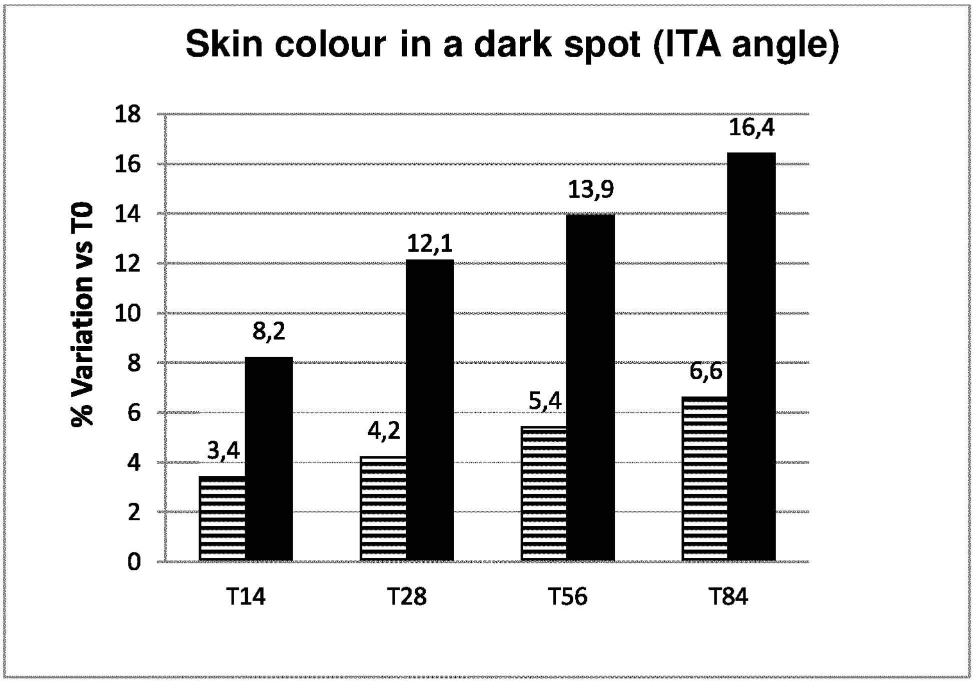


Figure 17

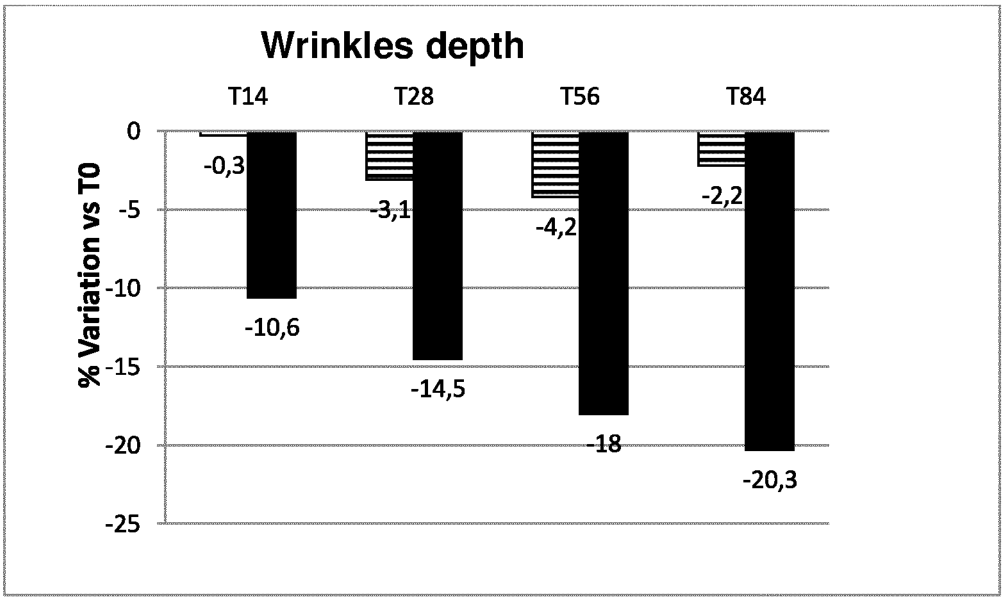

Figure 18

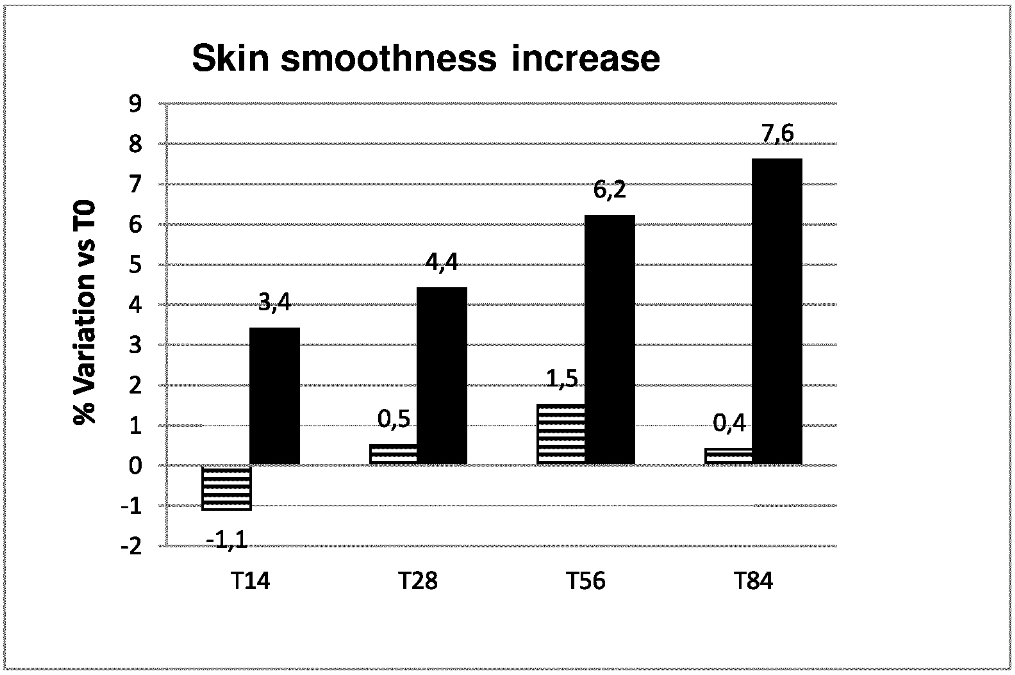


Figure 19

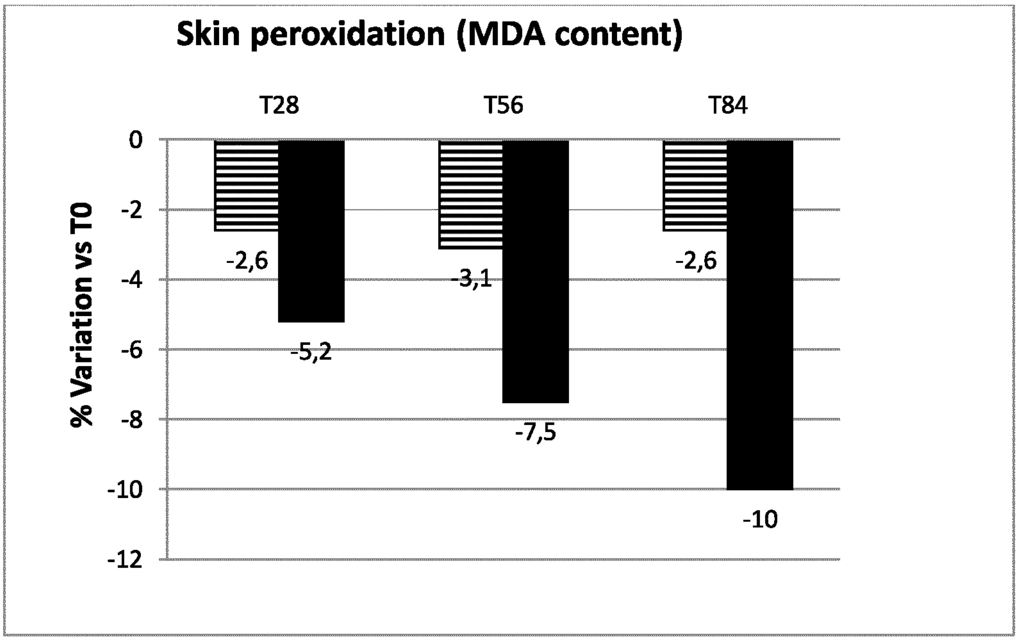

Figure 20

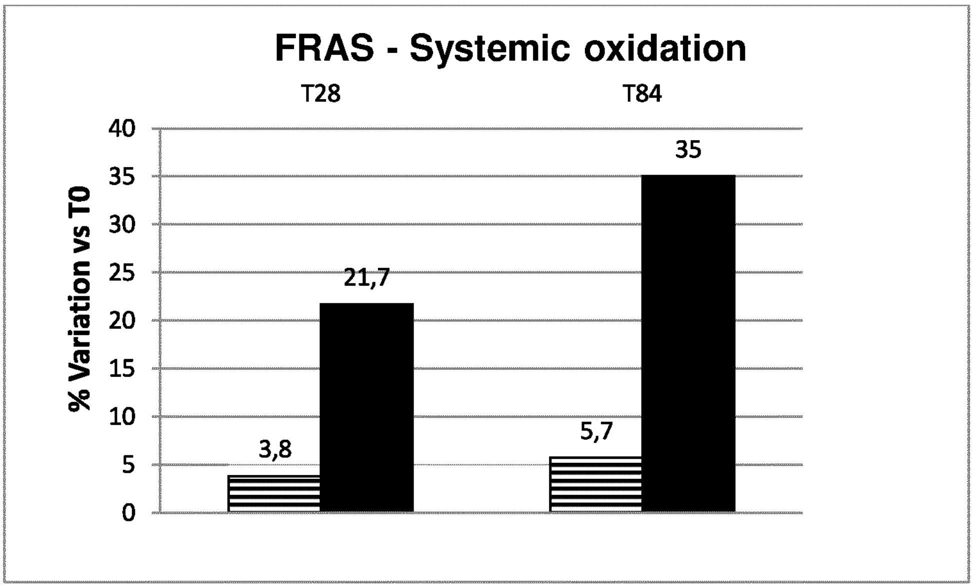
FRAS - Systemic oxidation
T28
T84
% Variation vs T0
40
35
30
25
20
15
10
5
0
3,8
21,7
5,7
35

# INTERNATIONAL SEARCH REPORT

International application No.
PCT/ES2019/070280

A. CLASSIFICATION OF SUBJECT MATTER

**See extra sheet**

According to International Patent Classification (IPC) or to both national classification and IPC

B. FIELDS SEARCHED

Minimum documentation searched (classification system followed by classification symbols)
A61K, A61Q

Documentation searched other than minimum documentation to the extent that such documents are included in the fields searched

Electronic data base consulted during the international search (name of data base and, where practicable, search terms used)

INVENES, EPODOC, BIOSIS, MEDLINE, EMBASE, INTERNET

C. DOCUMENTS CONSIDERED TO BE RELEVANT

| Category* | Citation of document, with indication, where appropriate, of the relevant passages | Relevant to claim No. |
|---|---|---|
| A | WO 2004069218 A1 (ROCHER YVES BIOLOG VEGETALE ET AL.) 19/08/2004, claims, page 6 | 1-24 |
| A | WO 2006041526 A1 (ACCESS BUSINESS GROUP INT LLC ET AL.) 20/04/2006, claims, paragraph [0040] | 1-24 |
| A | FR 2864785 A1 (OREAL) 08/07/2005, claims | 1-24 |
| A | WO 2013091684 A1 (ORIFLAME RES AND DEV LTD ET AL.) 27/06/2013, claims | 1-24 |

☐ Further documents are listed in the continuation of Box C. ☒ See patent family annex.

* Special categories of cited documents:
"A" document defining the general state of the art which is not considered to be of particular relevance.
"E" earlier document but published on or after the international filing date
"L" document which may throw doubts on priority claim(s) or which is cited to establish the publication date of another citation or other special reason (as specified)
"O" document referring to an oral disclosure use, exhibition, or other means.
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other documents , such combination being obvious to a person skilled in the art
"&" document member of the same patent family

| Date of the actual completion of the international search | Date of mailing of the international search report |
|---|---|
| 06/08/2019 | **(07/08/2019)** |
| Name and mailing address of the ISA/<br><br>OFICINA ESPAÑOLA DE PATENTES Y MARCAS<br>Paseo de la Castellana, 75 - 28071 Madrid (España)<br>Facsimile No.: 91 349 53 04 | Authorized officer<br>B. Pérez Esteban<br><br>Telephone No. 91 3498484 |

Form PCT/ISA/210 (second sheet) (January 2015)

**INTERNATIONAL SEARCH REPORT**

International application No. PCT/ES2019/070280

**Box No. II Observations where certain claims were found unsearchable (Continuation of item 2 of first sheet)**

This international search report has not been established in respect of certain claims under Article 17(2)(a) for the following reasons:

1. ☒ Claims Nos.: **n[os]: 1-10, 12-14, 21-24**
because they relate to subject matter not required to be searched by this Authority, namely:
Claims 1-10, 12-14, 21-24 relate to a subject matter that is considered by this International Searching Authority to be affected by PCT Rule 67.1(iv), concerning a method for treatment of the human or animal body by therapy. Nevertheless, a search has been carried out in respect of these claims on the basis of the alleged effects of the composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

## INTERNATIONAL SEARCH REPORT

Information on patent family members

International application No.
PCT/ES2019/070280

| Patent document cited in the search report | Publication date | Patent family member(s) | Publication date |
|---|---|---|---|
| WO2004069218 A1 | 19.08.2004 | FR2850572 A1 | 06.08.2004 |
| | | FR2850572 B1 | 01.04.2005 |
| WO2006041526 A1 | 20.04.2006 | US2014186433 A1 | 03.07.2014 |
| | | US8916180 B2 | 23.12.2014 |
| | | TW200611711 A | 16.04.2006 |
| | | TWI352600B B | 21.11.2011 |
| | | US2010285080 A1 | 11.11.2010 |
| | | US8697099 B2 | 15.04.2014 |
| | | US2007254021 A1 | 01.11.2007 |
| | | US7758878 B2 | 20.07.2010 |
| | | US2006257386 A1 | 16.11.2006 |
| | | US7959953 B2 | 14.06.2011 |
| | | US2006257509 A1 | 16.11.2006 |
| | | JP2008518042 A | 29.05.2008 |
| | | JP2007508320 A | 05.04.2007 |
| FR2864785 A1 | 08.07.2005 | NONE | |
| WO2013091684 A1 | 27.06.2013 | MX2014007495 A | 08.05.2015 |
| | | MX352165 B | 13.11.2017 |
| | | CL2014001661 A1 | 06.03.2015 |
| | | EA201490613 A1 | 30.12.2014 |
| | | EA031552 B1 | 31.01.2019 |
| | | CN104254370 A | 31.12.2014 |
| | | EP2886163 A1 | 24.06.2015 |
| | | EP2862600 A1 | 22.04.2015 |
| | | US2014301961 A1 | 09.10.2014 |
| | | EP2729220 A1 | 14.05.2014 |
| | | EP2729220 B1 | 04.11.2015 |

Form PCT/ISA/210 (patent family annex) (January 2015)

## INTERNATIONAL SEARCH REPORT

International application No.

PCT/ES2019/070280

### CLASSIFICATION OF SUBJECT MATTER

***A61K8/97*** (2017.01)
***A61Q17/00*** (2006.01)
***A61Q19/00*** (2006.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KRUTMANN J. et al.** Environmentally induced (extrinsic) skin aging. *Hautarzt,* February 2016, vol. 67 (2), 99-102
- **MORGENSTERN, V. et al.** Atopic diseases, allergic sensitization, and exposure to traffic-related air pollution in children. *Am. J. Respir. Crit. Care Med.,* 2008, vol. 177, 1331-1337
- **SONG, S. et al.** Acute health effects of urban fine and ultrafine particles on children with atopic dermatitis. *Environ. Res.,* 2011, vol. 111, 394-399
- **KIM, J. et al.** Symptoms of atopic dermatitis are influenced by outdoor air pollution. *J. Allergy Clin. Immunol.,* 2013, vol. 132, 495-499
- **VAN DEN BOGAARD, E.H. et al.** Coal tar induces AHR-dependent skin barrier repair in atopic dermatitis. *J. Clin. Invest.,* 2013, vol. 123, 917-927
- **KIM, H.O. ; KIM, J.H. ; CHUNG, B.Y. ; CHOI, M.G. ; PARK, C.W.** Increased expression of the aryl hydrocarbon receptor in patients with chronic inflammatory skin diseases. *Exp. Dermatol.,* 2014, vol. 23, 278-281
- **DIMEGLIO, P. et al.** Activation of the aryl hydrocarbon receptor dampens the severity of inflammatory skin conditions. *Immunity,* 2014, vol. 40, 989-1001
- **JEAN KRUTMANN ; DOMINIQUE MOYAL ; WEI LIU ; SANJIV KANDAHARI ; GEUN-SOO LEE ; NOPPAKUN NOPADON ; LEIHONG FLORA XIANG ; SOPHIE SEITÉ.** Pollution and acne: is there a link?. *Clinical, Cosmetic and Investigational Dermatology,* May 2017
- **JU Q ; ZOUBOULIS CC ; XIA L.** Environmental pollution and acne: Chloracne. *Dermato-endocrinology,* 2009, vol. 1 (3), 125-128
- **VIERKOLER A et al.** Airborne particle exposure and extrinsic skin aging. *J. Invest. Dermatol.,* 2010, vol. 130 (12), 2719-2726
- **HÜLS A et al.** Traffic-Related Air Pollution Contributes to Development of Facial Lentigines: Further Epidemiological Evidence from Caucasians and Asians. *J. Invest. Dermatol.,* May 2016, vol. 136 (5), 1053-6
- Quercetin Is More Effective than Cromolyn in Blocking Human Mast Cell Cytokine Release and Inhibits Contact Dermatitis and Photosensitivity in Humans. **WENG, ZUYI et al.** PLoS ONE. 2012, vol. 7.3, e33805
- **SIM, H.S.S. ; S.S.** Acetoside inhibits a-MSH- induced melanin production in B16 melanoma cells by inactivation of adenyl cyclase. *Journal of Pharmacy and Pharmacology,* 2009, vol. 61, 1347-1351
- **YOUNG-OK SONA, S.-A.L.E.A.** Acteoside inhibits melanogenesis in B16F10 cells through ERK activation and tyrosinase down-regulation. *Journal of Pharmacy and Pharmacology,* 2011, vol. 63, 1309-1319
- **MANCEBO SE ; WANG SQ.** Recognizing the impact of ambient air pollution on skin health. *J Eur Acad Dermatol Venereol.,* December 2015, vol. 29 (12), 2326-32
- **MULERO-NAVARRO S ; FERNANDEZ-SALGUERO PM.** New Trends in Aryl Hydrocarbon Receptor Biology. *Frontiers in Cell and Developmental Biology*
- **ABEL J ; HAARMANN-STEMMANN T.** An introduction to the molecular basics of aryl hydrocarbon receptor biology. *Biol Chem.,* November 2010, vol. 391 (11), 1235-48
- **KRUTMANN J ; JUX B ; LUECKE S ; FRITSCHE E ; ABEL J ; ESSEL C ; RANNUG A.** Involvement of arylhydrocarbon receptor (AhR-) signaling in skin melanogenesis. *J Invest Dermatol,* 2008, vol. 128, 22
- **MORITA A ; TORII K ; MAEDA A ; YAMAGUCHI Y.** Molecular basis of tobacco smoke-induced premature skin aging. *J Investig Dermatol Symp Proc.,* August 2009, vol. 14 (1), 53-5
- **MURPHY KA ; VILLANO CM ; DORN R ; WHITE LA.** Interaction between the aryl hydrocarbon receptor and retinoic acid pathways increases matrix metalloproteinase-1 expression in keratinocytes. *J Biol Chem.,* 11 June 2004, vol. 279 (24), 25284-93
- **LUECKE S ; BACKLUND M ; JUX B ; ESSER C ; KRUTMANN J ; RANNUG A.** The aryl hydrocarbon receptor (AHR), a novel regulator of human melanogenesis. Pigment. *Cell Melanoma Res.,* December 2010, vol. 23 (6), 828-33
- **AFAQ F ; ZAID MA ; PELLE E ; KHAN N ; SYED DN ; MATSUI MS ; MAES D ; MUKHTAR H.** Aryl hydrocarbon receptor is an ozone sensor in human skin. *J Invest Dermatol.,* October 2009, vol. 129 (10), 2396-403

- **ONO Y ; TORII K ; FRITSCHE E ; SHINTANI Y ; NISHIDA E ; NAKAMURA M ; SHIRAKATA Y ; HAARMANN-STEMMANN T ; ABEL J ; KRUTMANN J.** Role of the aryl hydrocarbon receptor in tobacco smoke extract-induced matrix metalloproteinase-1 expression. *Exp. Dermatol.,* May 2013, vol. 22 (5), 349-53
- **HIDAKA T ; OGAWA E ; KOBAYASHI EH ; SUZUKI T ; FUNAYAMA R ; NAGASHIMA T ; FUJIMURA, T ; AIBA S ; NAKAYAMA K ; OKUYAMA R.** The aryl hydrocarbon receptor AhR links atopic dermatitis and air pollution via induction of the neurotrophic factor artemin. *Nat Immunol.,* January 2017, vol. 18 (1), 64-73
- **JANERO D.R.** Malondialdehyde and thiobarbituric acid-reactivity as diagnostic indices of lipid peroxidation and peroxidative tissue injury. *Free Radic Biol Med.,* 1990, vol. 9 (6), 515-40
- **HAARMANN-STEMMANN T ; ABEL J ; FRITSCHE E ; KRUTMANN J.** The AhR-Nrf2 pathway in keratinocytes: on the road to chemoprevention. *J Invest Dermatol.,* January 2012, vol. 132 (1), 7-9
- **CHAMPION S. et al.** Activation of the NFκB Pathway Enhances AhR Expression in Intestinal Caco-2 Cells. *ISRN Toxicology,* 2013, vol. 2013
- **TAUCHI et al.** Constitutive Expression of Aryl Hydrocarbon Receptor in Keratinocytes Causes Inflammatory Skin Lesions. *Molecular and cellular biology,* November 2005, 9360-9368
- **FURUE et al.** Role of AhR/ARNT system in skin homeostasis. *Arch Dermatol Res.,* 2014, vol. 306, 769-779